# EUROPEAN PATENT SPECIFICATION

(11) **EP 2 051 996 B1**
(45) Date of publication and mention of the grant of the patent: **10.12.2014**
(21) Application number: 07763740.3
(22) Date of filing: 05.07.2007
(51) Int. Cl.: C07K 14/725

(54) **METHOD FOR ENGINEERING T-CELL RECEPTORS**
VERFAHREN FÜR DIE ENTWICKLUNG VON T-ZELL-REZEPTOREN
PROCÉDÉ POUR SYNTHÉTISER PAR GÉNIE GÉNÉTIQUE DES RÉCEPTEURS DE LYMPHOCYTES T

(30) Priority: 05.07.2006 AT 11462006
(43) Date of publication of application: 29.04.2009
(60) Divisional application: 14191631.2
(73) Proprietor: F-Star Biotechnologische Forschungs- und Entwicklungsges.m.b.H, 1030 Vienna (AT)
(72) Inventor: HIMMLER, Gottfried, A-2301 Gross-Enzersdorf (AT); RÜKER, Florian, A-1170 Vien (AT)
(74) Representative: Walton, Seán Malcolm
(86) International application number: PCT/AT2007/000342
(87) International publication number: WO 2008/003115

(56) References cited:
- WO-A-2004/074322
- WO-A-2006/072620
- WEBER K SCOTT ET AL: "Class II-restricted T cell receptor engineered in vitro for higher affinity retains peptide specificity and function" PROCEEDINGS OF THE NATIONAL ACADEMY OF SCIENCES OF THE UNITED STATES OF AMERICA, vol. 102, no. 52, December 2005 (2005-12), pages 19033-19038, XP002449077 ISSN: 0027-8424
- REITER Y ET AL: "CONSTRUCTION OF A FUNCTIONAL DISULFIDE-STABILIZED TCR FV INDICATES THAT ANTIBODY AND TCR FV FRAMEWORKS ARE VERY SIMILAR IN STRUCTURE" IMMUNITY, CELL PRESS, US, vol. 2, no. 3, March 1995 (1995-03), pages 281-287, XP009004075 ISSN: 1074-7613
- LAUGEL BRUNO ET AL: "Design of soluble recombinant T cell receptors for antigen targeting and T cell inhibition" JOURNAL OF BIOLOGICAL CHEMISTRY, vol. 280, no. 3, 21 January 2005 (2005-01-21), pages 1882-1892, XP002449078 ISSN: 0021-9258
- SIMON T ET AL: "A FUNCTIONAL ANTIBODY MUTANT WITH AN INSERTION IN THE FRAMEWORK REGION 3 LOOP OF THE V-H DOMAIN IMPLICATIONS FOR ANTIBODY ENGINEERING" PROTEIN ENGINEERING, vol. 5, no. 3, 1992, pages 229-234, XP008083103 ISSN: 0269-2139
- BOULTER JONATHAN M ET AL: "Stable, soluble T-cell receptor molecules for crystallization and therapeutics" PROTEIN ENGINEERING, OXFORD UNIVERSITY PRESS, SURREY, GB, vol. 16, no. 9, September 2003 (2003-09), pages 707-711, XP002275339 ISSN: 0269-2139
- MOLLOY P E ET AL: "Soluble T cell receptors: novel immunotherapies" CURRENT OPINION IN PHARMACOLOGY, ELSEVIER SCIENCE PUBLISHERS,, NL, vol. 5, no. 4, August 2005 (2005-08), pages 438-443, XP004969013 ISSN: 1471-4892
- BOULTER J M ET AL: "Stable, soluble, high-affinity, engineered T cell receptors: novel antibody-like proteins for specific targeting of peptide antigens" CLINICAL AND EXPERIMENTAL IMMUNOLOGY, vol. 142, no. 3, December 2005 (2005-12), pages 454-460, XP002449079 ISSN: 0009-9104
- HOLLER P D ET AL: "In vitro evolution of a T cell receptor with high affinity for peptide/MHC.", PROCEEDINGS OF THE NATIONAL ACADEMY OF SCIENCES OF THE UNITED STATES OF AMERICA 9 MAY 2000, vol. 97, no. 10, 9 May 2000 (2000-05-09), pages 5387-5392, ISSN: 0027-8424
- SHUSTA E V ET AL: "Directed evolution of a stable scaffold for T-cell receptor engineering.", NATURE BIOTECHNOLOGY JUL 2000, vol. 18, no. 7, July 2000 (2000-07), pages 754-759, ISSN: 1087-0156

## Description

### Field of the invention

The present invention relates to a novel method for engineering modified T-cell receptors and T-cell receptor domain polypeptides with the aim to impart them with specific binding properties. Further, modified T-cell receptor domain polypeptides obtained by said method and their use for establishing libraries and developing detection and screening methods for possible binding structures are disclosed.

### Background:

T-cell receptors (TCRs) are important molecules of the immune system. Its extracellular domains are homologous with and structurally similar to an antibody Fab fragment.

T-cell receptors are expressed in nature on the surface of T-cells usually as alpha/beta and gamma/delta heterodimeric integral membrane proteins, each subunit comprising a short intracellular segment, a single transmembrane alpha-helix and two globular extracellular Ig-superfamily domains. The TCR-heterodimer is stabilized by an extracellular, membrane proximal, inter-chain disulphide bond (Immunobiology. 5th ed. Janeway, Charles A.; Travers, Paul; Walport, Mark; Shlomchik, Mark. New York and London: Garland Publishing; 2001).

TCRs therefore have four extracellular domains, the two membrane proximal (C-terminal) domains, which are constant, and the two N-terminal domains, which are variable. The variable domains are encoded by variable gene segments which are rearranged with junctional and constant gene segments (and diversity gene segments in the case of β-chains) to produce the TCR diversity observed in the mature immune system.

Both variable domains as well as constant domains of T-cell receptors are structurally similar to antibody domains and exhibit the typical "immunoglobulin fold":

Each domain has a similar structure of two beta sheets packed tightly against each other in a compressed antiparallel beta barrel.

The immunoglobulin fold of TCR-constant domains (C-domains) contains a 3-stranded sheet packed against a 4-stranded sheet. The fold is stabilized by hydrogen bonding between the beta-strands of each sheet, by hydrophobic bonding between residues of opposite sheets in the interior, and by a disulfide bond between the sheets. The 3-stranded sheet strands are denoted C, F, and G, and the 4-stranded sheet comprises the strands A B, D, and E. The letters A through G denote the sequential positions of the beta strands along the amino acid sequence of the immunoglobulin fold.

The fold of variable domains (V-domains) has 9 beta strands arranged in two sheets of 4 and 5 strands. The 5-stranded sheet is structurally homologous to the 3-stranded sheet of constant domains, but contains the extra strands C' and C". The remainder of the strands (A, B, C, D, E, F, G) have the same topology and similar structure as their counterparts in constant domain immunoglobulin folds.

A disulfide bond links strands B and F in opposite sheets, as in constant domains.

All numbering of the amino acid sequences and designation of protein loops and sheets of the TCRs is according to the IMGT numbering scheme (IMGT, the international ImMunoGeneTics information system@imgt.cines.fr; http://imgt.cines.fr; Lefranc et al., (2003) Dev Comp Immunol 27:55 77.; Lefranc et al. (2005) Dev Comp Immunol 29:185-203).

The variable domains of both TCR chains contain three hypervariable loops, or complementarity-determining regions (CDRs). The three CDRs of a V-domain (CDR1, CDR2, CDR3) cluster at one end of the beta barrel. The CDRs are loops that connect beta strands B-C, C'-C", and F-G of the immunoglobulin fold. The residues in the CDRs vary from one TCR molecule to the next, imparting antigen specificity to each TCR.

The V-domains at the tips of TCR-molecules are closely packed such that the 6 CDRs (3 on each variable domain) cooperate in constructing a surface (or cavity) for antigen-specific binding. The natural antigen binding site of a TCR thus is composed of the loops which connect strands B-C, C'-C", and F-G of the light chain variable domain and strands B-C, C'-C", and F-G of the heavy chain variable domain. The extent of the specific contribution to antigen binding by the six CDR loops can vary between different TCRs.

It has been demonstrated that T-cell receptors have therapeutic and diagnostic potential and can be manipulated similarly to antibody molecules (e.g. Molloy et al. Curr Opin Pharmacol. (2005) 5:438-443; Boulter & Jakobsen (2005) Clin Exp Immunol. 142:454-460; Simon et al (1992) Protein Engineerings 5: 229-234).

Cloning and expression of soluble and cell anchored T-cell receptors in various formats has been demonstrated (e.g. Moysey et al. (2004) Anal Biochem. 326:284-286; Wulfing & Plueckthun (1994) J Mol Biol.242:655-669; Boulter et al. (2003) Protein Eng. 16:707-711; Schodin et al. (1996) Mol Immunol 33:819 829; Chung et al. (1994) Proc Natl Acad Sci USA 91:12654 12658; Plaksin et al. (1997) J Immunol 158:2218 2227; Willcox et al. (1999) Protein Sci 8:2418 2423; Weber et al. (2005) Proc Natl Acad Sci U S A. 102:19033-19038; WO04050705A2; WO9618105A1; WO04033685A1; WO02066636A2). WO02059263C2 describes transgenic animals comprising a humanized immune system to develop human TCR molecules.

The generation of high affinity binding has been shown and is being pursued similar to antibody affinity maturation technologies (e.g. Boulter et al. Nat Biotechnol. (2005) 23:349-354; Chlewicki et al. (2005) J Mol Biol. 346:223-239;

Laugel et al. J.Biol. Chemistry 280(3) 1882-1892 (2005); Monza et al. PNAS 103(26)9867-9872 (2006);

Shusta et al. (2000) 18:754-759; Holler et al. (2000) Proc Natl Acad Sci USA 97:5387 92). WO04044004A2, WO05116646A1 and WO9839482A1 describe ribosome and phage display of TCR chains and methods to select for TCR molecules against specific antigens. WO0148145A2 describes high affinity TCRs. Manipulation of the extracellular variable domains of T-cell receptors has been performed for the purpose of specificity engineering via modification of the CDR-regions (WO05114215A2; WO0155366C2).

The CDR-loops of a TCR variable domain define the antigen specificity. The rest of the domain is termed framework (FR). These framework regions are composed of beta-strand and loop structures.

The loops which are not CDR-loops in a native TCR domain do not have antigen-binding or epitope-binding specificity but contribute to the correct overall folding of the TCR-domain and consequently also to the correct positioning of the CDRs and to interaction between domains. These loops are named structural loops for the purpose of this invention.

The framework regions of TCR domains have been modified e.g for stabilization of the dimers. WO06037960A2 and WO06056733A1 describe the introduction of a non-native disulfide interchain bond. WO0157211A1 describes heterodimerization of TCR chains by fusion to leucine-zipper peptides.

EP0640130B1 describes chimeric immunoglobulin superfamily protein analogues (chi-proteins) having more than one biological binding site (single V domains or Fvs). The binding sites on these proteins are comprised of hypervariable regions derived from molecules related to the immunoglobulin superfamily of molecules, including immunoglobulins, cell surface antigens (such as T-cell antigens) and cell receptors (such as Fc-receptor). The hypervariable regions are called "CDR-like regions" and define ligand binding sites. Additionally, the chi-protein has at least one more ligand binding site segment, also a CDR-like region, spliced into the FR-like regions of the beta-barrel domain.

Each ligand binding site of the chi-protein therefore is comprised of a CDR-like region derived from molecules of the immunoglobulin superfamily. For example, a ligand binding site is comprised of the CDRs derived from an immunoglobulin molecule whose ligand is an antigen.

EP0640130B1 thus teaches how to splice CDR-like regions with a given specificity from a molecule of the immunoglobulin superfamily into the structural loops of a variable domain.

It is postulated by the inventors of the chi-proteins that functional bispecific antibodies can be prepared by this technique. However, there is a requirement for this technique that the relative orientations of the CDR-like loops (CDR loop symmetry) for a variable domain be reproduced to a reasonable approximation in the relative orientation of the structural loops. EP0640130B1 claims that such an approximation of the CDR-like loop symmetry does exist for the structural loops. However, it is doubtful that the relative orientation of the CDR-loops and the structural loops is similar in sufficient detail and resolution; consequently it has not been described to date that it is actually possible to develop bispecific molecules by this technique.

EP0640130B1 exemplifies that R19.9 (a monoclonal murine antibody specific for the p-azobenzenearsonate) and 26-10 (monoclonal murine antibody specific for ouabain) were used as the framework providing the primary CDR loops respectively, and the CDR-loops of murine anti-lysozyme antibody D1.3 were grafted into the structural loop regions. However, functional specificity after grafting is not described.

Another example describes that the single chain antibody 26-10 specific for ouabain could retain its ouabain-specificity after grafting two CDRs from the lysozyme-specific antibody into the structural loops of the ouabain-specific single-chain Fv anti-body fragment. However, it is not described that the antibody fragment which was made according to this method had also ly-sozyme-binding specificity.

In order to provide additional functions to TCR molecules various fusion molecules have been designed: Mosquera et al. (2005) J Immunol 174:4381 4388 describes a novel antibody-like single-chain TCR human IgG1 fusion protein; Epel et al. (2002) Cancer Immunol Immunother 51:565 573 describes a functional recombinant single-chain T cell receptor fragment fused to exotoxin A protein, capable of selectively targeting antigen-presenting cells. Fusion of single-chain TCR to IL-2 has been reported to reduce lung metastases in a transgenic mouse tumor model (Card et al. (2004) Cancer Immunol Immunother 53:345 357). WO06054096A2 describes a soluble bifunctional protein comprising an association between a T cell receptor (TCR) and a superantigen.

It is of great value for therapeutic, diagnostic and analytical applications to introduce additional functions to TCR molecules. The generation of fusion proteins is one approach to reach this goal. Fusion proteins, however, are frequently difficult to produce and may lead to enhanced immunogenicity if the molecule is used therapeutically. Grafting of CDR-loops into structural loop regions of variable domains has not been demonstrated to allow for engineering of bispecific molecules.
The present invention provides a solution for the introduction of new functions into TCR molecules.

### Brief description of the invention:

The present invention advantageously provides a method for engineering a T-cell receptor domain polypeptide comprising a structural loop region to introduce a new antigen binding site into said structural loop region of said T-cell receptor domain polypeptide, wherein the structural loop is a non-CDR loop, and determining the binding of said T-cell receptor domain polypeptide to an epitope of an antigen, wherein the unmodified T-cell receptor domain polypeptide does not significantly bind to said epitope of said antigen comprising the steps of:
- providing a nucleic acid encoding a T-cell receptor domain polypeptide comprising at least one structural loop region,
- modifying at least one nucleotide residue of at least one of said structural loop regions by site-directed random mutation, wherein two or more specific amino acid residues of the loops are exchanged or introduced using randomly generated inserts into such structural loops,
- transferring said modified nucleic acid in an expression system,
- expressing said modified T-cell receptor domain polypeptide,
- contacting the expressed modified T-cell receptor domain polypeptide with said epitope, and
- determining whether said modified T-cell receptor domain polypeptide binds to said epitope.

According to the invention the so obtained T-cell receptor domain polypeptides can bind specifically to one single epitope but also to two or more epitopes. The T-cell receptor domain polypeptides according to the disclosure can be of human or animal origin, preferably of human or murine origin. The inventive T-cell receptor domains can be selected from the variable or constant domains, preferably from V-alpha, V-beta, V-gamma, V-delta, C-alpha, C-beta, C-gamma, C-delta domains.

According to a specific embodiment of the invention the modified loop regions of the variable domains can comprise at least one modification within amino acids 11 to 19, amino acids 43 to 51, amino acids 67 to 80 or amino acids 90 to 99. The modified loop regions of the constant domains can comprise at least one modification within amino acids 9 to 20, amino acids 27 to 36, amino acids 41 to 78, amino acids 82 to 85, amino acids 90 to 102 or amino acids 107 to 116. The numbering of the amino acid positions of the domains is that of the IMGT.

>

The method according to the invention provides for example a modification of at least one nucleotide of a nucleic acid resulting in a substitution, deletion and/or insertion of one or more amino acids of the T-cell receptor domain polypeptide encoded by said nucleic acid.

Alternatively, at least one amino acid of at least one structural loop region is modified by site-directed random mutation, wherein the randomly modified nucleic acid molecule can comprise at least one nucleotide repeating unit having the coding sequence NNS, NNN, NNK, TMT, WMT, RMC, RMG, MRT, SRC, KMT, RST, YMT, MKC, RSA, RRC, where the coding is according to IUPAC.

Also described herein are T-cell receptor domain polypeptides obtainable by this method and their use for the preparation of a protein libraries of variant T-cell receptor domain polypeptides.

Also described herein are Also described herein are libraries comprising at least 10 T-cell receptor domain polypeptides or T-cell receptors with a modification in at least one structural loop region, alternatively with mutations of at least 3 amino acid positions in at least one structural loop region. The T-cell receptor domain polypeptides can be V-alpha, V-beta, V-gamma, V-delta, C-alpha, C-beta, C-gamma or C-delta.

Also described herein is a method for specifically binding and/or detecting a molecule is provided, comprising the steps of (a) contacting a library of modified T-cell receptors or modified T-cell receptor domain polypeptides obtainable by the inventive method with a test sample containing said molecule, and optionally (b) detecting the potential formation of a specific T-cell receptor/molecule complex.

Also described herein is a method for specifically isolating a modified T-cell receptor binding to a molecule comprising the steps of (a) contacting a library of modified T-cell receptors or a modified T-cell receptor obtainable by a method according to the invention with a sample containing said molecule,(b) separating the specific modified T-cell receptor/molecule complex formed, and (c) optionally isolating the modified T-cell receptor from said complex.

A kit of binding partners is also described herein containing (a) a library of modified T-cell receptors or modified T-cell receptor domain polypeptides obtained by the inventive method and (b) a binding molecule containing an epitope of an antigen.

The binding molecule of the kit can be used for selecting a modified T-cell receptor domain polypeptide from a library described herein.

Also described herein is a T-cell receptor domain polypeptide comprising at least one structural loop region said at least one loop region comprising at least one modification enabling a binding of said at least one modified loop region to an epitope of an antigen wherein the unmodified T-cell receptor domain polypeptide does not bind to said epitope.

Said T-cell receptor domain polypeptide can preferably bind to an epitope of an antigen, for example serum proteins, Fc-receptors, complement molecules and serum albumins. Binding to these antigens can be advantageous as native TCRs do not have any effector functions. By developing modified T-cell receptor domain polypeptides binding Fc receptors like Fc gamma I, II or III or neonatal Fc receptors or complement proteins, TCRs can be obtained having effector function capabilities. Alternatively, also serum half lifesof the so modified TCRs due to binding to neonatal Fc receptors can be increased if appropriate. This can be especially highly advantageous for therapeutic purposes.

The modified T-cell receptor domain polypeptides can also contain at least two modified structural loop regions.

Additionally, the T-cell receptor comprising at least one modified T-cell receptor domain as described above can be V-alpha, V-beta, V-gamma, V-delta, C-alpha, C-beta, C-gamma, C-delta or a part thereof and said at least one modified structural loop region can comprise at least 3 amino acid modifications.

Also described herein is a molecule or composition comprising at least one modified T-cell receptor domain and at least one other binding molecule, wherein said other binding molecule is selected from the group of modified T-cell receptor domains according to, immunoglobulins, soluble receptors, ligands, nucleic acids, and carbohydrates. Said molecule or composition can be further characterized in that the modified loop regions of a V-alpha, V-beta, V-gamma, V-delta comprise at least one modification within amino acids 11 to 19, amino acids 43 to 51, amino acids 67 to 80 or amino acids 90 to 99, where the numbering of the amino acid position of the domains is that of the IMGT.

Alternatively, said molecule or composition can be characterised in that the loop regions of a C-alpha, C-beta, C-gamma, C-delta comprise at least one modification within amino acids 9 to 20, amino acids 27 to 36, amino acids 41 to 78, amino acids 82 to 85, amino acids 90 to 102 or amino acids 107 to 116, where the numbering of the amino acid position of the domains is that of the IMGT.

Also described herein is a nucleic acid encoding said T-cell receptor domain or part thereof.

### Detailed description of the invention:

A "structural loop" or "non-CDR-loop" according to the present invention is to be understood in the following manner: T-cell receptors are made of domains with a so called immunoglobulin fold. In essence, anti-parallel beta sheets are connected by loops to form a compressed antiparallel beta barrel. In the variable region, some of the loops of the domains contribute essentially to the specificity of the TCR, i.e. the binding to an antigen. These loops are called CDR-loops. All other loops of antibody variable domains are rather contributing to the structure of the molecule and/or interaction with other domains. These loops are defined herein as structural loops or non-CDR-loops.

T-cell receptors are molecules on T-cells, for the purpose of this invention it also includes molecules derived from T-cell receptors including but not limited to fusion proteins, chimera of T-cell receptor sequences with other immunoglobulin domain sequences, chimera of T-cell receptor sequences of one species with sequences of different species; soluble T-cell receptors, single-chain T-cell receptors, zipper-dimerized T-cell receptors.

A TCR-domain polypeptide is a stretch of amino acids derived from a TCR variable or constant domain comprising at least one structural loop.

The term immunoglobulin herein is used for antibodies, fragments thereof, variable regions, constant regions and fusion molecules thereof

In particular, the present invention relates to a method for engineering a T-cell receptor domain polypeptide binding specifically with its modified structural loops to an epitope of an antigen selected from the group consisting of allergens, tumor associated antigens, self antigens, enzymes, Fc-receptors, proteins of the complement system, serum molecules, bacterial antigens, fungal antigens, protozoan antigen and viral antigens.

In a preferred embodiment the T-cell receptor domain polypeptide is binding with its modified structural loops specifically to at least two such epitopes that differ from each other, either of the same antigen or of different antigens.

It is understood that the term "T-cell receptor domain polypeptide", "modified T-cell receptor domain" or "T-cell receptor domain according to the invention" includes a derivative of a T-cell receptor as well. A derivative is any combination of one or more T-cell receptor domains of the invention and/or a fusion protein in which any T-cell receptor domain of the invention may be fused at any position of one or more other proteins, including, but not limited to other T-cell receptor domains, immunoglobulin domains, Fc parts, ligands, scaffold proteins, enzymes, toxins, serum proteins and the like. A derivative of the T-cell receptor of the invention may also be obtained by connecting the T-cell receptor domain polypeptide of the invention to other substances by various chemical techniques such as covalent coupling, electrostatic interaction, disulphide bonding etc. The other substances bound to the T-cell receptor domain polypeptide may be lipids, carbohydrates, nucleic acids, organic and inorganic molecules or any combination thereof (e.g. PEG, pro-drugs or drugs). A derivative is also a T-cell receptor domain polypeptide with the same amino acid sequence but made completely or partly from non-natural or chemically modified amino acids.

The engineered molecules will be useful as stand-alone proteins as well as fusion proteins or derivatives, most typically fused in such a way as to be part of larger T-cell receptor structures or complete T-cell receptor molecules, or parts thereof such as bispecific and multispecific single-chain T-cell receptors or combined formulations wherein the engineered polypeptides are combined as needed. It will be possible to use the engineered proteins to produce molecules which are monospecific, bispecific, trispecific, and may even carry more specificities at the same time, and it will be possible to control and preselect the valency of binding at the same time according to the requirements of the planned use of such molecules.

According to the present invention, binding regions to antigens or antigen binding sites to all kinds of allergens, tumor associated antigens, self antigens, enzymes, Fc-receptors, proteins of the complement system, serum molecules, bacterial antigens, fungal antigens, protozoan antigens and viral antigens, may be introduced into a structural loop region of a given T-cell receptor domain structure. The antigens may be naturally occurring molecules or chemically synthesized molecules or recombinant molecules, all either in solution or on or in particles such as solid phases, on or in cells or on viral surfaces.

Preferred antigens are serum proteins, Fc-receptors, like Fc gamma I, II or II, neonatal Fc receptors, complement molecules and serum albumins.

The term "allergens, tumor associated antigens, self antigens, enzymes, Fc-receptors, proteins of the complement system, serum molecules, bacterial antigens, fungal antigens, protozoan antigen and viral antigens" shall include all allergens and antigens and targets capable of being recognised by a T-cell receptor or antibody, and fragments of such molecules as well as small molecules such as haptens.
The term "epitope" shall mean a molecular structure which may completely make up a specific binding partner or be part of a specific binding partner to the binding domain or the T-cell receptor domain polypeptide.

Chemically, an epitope may either be composed of a carbohydrate, a peptide, a fatty acid, an organic, biochemical or inorganic substance or derivatives thereof and any combinations thereof. If an epitope is a polypeptide, it will usually include at least 3 amino acids, preferably 8 to 50 amino acids, and more preferably between about 10-20 amino acids in the peptide. There is no critical upper limit to the length of the peptide, which could comprise nearly the full length of a polypeptide sequence. Epitopes can be either linear or conformational epitopes. A linear epitope is comprised of a single segment of a primary sequence of a polypeptide chain. Linear epitopes can be contiguous or overlapping. Conformational epitopes are comprised of amino acids brought together by folding of the polypeptide to form a tertiary structure and the amino acids are not necessarily adjacent to one another in the linear sequence.
Specifically, epitopes are at least part of diagnostically relevant molecules, i.e. the absence or presence of an epitope in a sample is qualitatively or quantitatively correlated to either a disease or to the health status of a patient or to a process status in manufacturing or to environmental and food status. Epitopes may also be at least part of therapeutically relevant molecules, i.e. molecules which can be targeted by the specific binding domain which changes the course of the disease.

Preferred "allergens, tumor associated antigens, self antigens, enzymes, Fc-receptors, proteins of the complement system, serum molecules, bacterial antigens, fungal antigens, protozoal antigen and viral antigens," are those allergens or antigens, which have already been proven to be or are capable of being immunologically or therapeutically relevant, especially those, for which a clinical efficacy has been tested.

According to another aspect of the present invention also other binding capacities may be introduced in the structural loop regions of T-cell receptor domain polypeptides, e.g. binding capacities for small molecules, for drugs or enzymes, catalytic sites of enzymes or enzyme substrates or the binding to a transition state analogue of an enzyme substrate.

Preferably the new antigen binding site in the structural loops is foreign to the unmodified T-cell receptor domain polypeptide. Thus targets like effector molecules, serum proteins or Fc-receptors and cell surface molecules are preferred as binding partners of the T-cell receptor domain polypeptide.

As used herein, the term "specifically binds" or "specific binding" refers to a binding reaction which is determinative of the cognate ligand of interest in a heterogeneous population of molecules. Thus, under designated conditions (e.g. immunoassay conditions), the specified T-cell receptor domain polypeptide binds to its particular "target" and does not bind in a significant amount to other molecules present in a sample.

The term "expression system" refers to nucleic acid molecules containing a desired coding sequence and control sequences in operable linkage, so that hosts transformed or transfected with these sequences are capable of producing the encoded proteins. In order to effect transformation, the expression system may be included on a vector; however, the relevant DNA may then also be integrated into the host chromosome. Alternatively, an expression system can be used for in vitro transcription/translation.

According to a preferred embodiment of the present invention the TCR domain polypeptide is of human or animal origin, preferably of camelid, rabbit, chicken, rat, dog, horse, sheep or murine origin.

Since the modified T-cell receptor polypeptide may be employed for various purposes, in particular in pharmaceutical compositions, the T-cell receptor domain polypeptide is preferably of human or murine origin. Of course, the modified T-cell receptor polypeptide may also be a humanized or a chimeric T-cell receptor domain polypeptide. In the most preferred embodiment of the invention the modified T-cell receptor domain polypeptide is of human origin or a humanized version of a T-cell receptor domain polypeptide of any species.

The structural loop regions of a T-cell receptor variable domain polypeptide are selected preferably from the structural loops that comprise amino acids 11 to 19, amino acids 43 to 51, amino acids 67 to 80 or amino acids 90 to 99, where the numbering of the amino acid position of the domains is that of the IMGT.

The structural loop regions of a T-cell receptor constant domain polypeptide are selected preferably from the structural loops that comprise amino acids 9 to 20, amino acids 27 to 36, amino acids 41 to 78, amino acids 82 to 85, amino acids 90 to 102 or amino acids 107 to 116, where the numbering of the amino acid position of the domains is that of the IMGT.

Preferably, the new antigen binding sites in the structural loops are introduced in the T-cell receptor domain polypeptides encoded by the selected nucleic acid by substitution, deletion and/or insertion of at least one nucleotide.

According to another preferred embodiment of the present invention the modification of at least one nucleotide in at least one structural loop region results in a substitution, deletion and/or insertion in the T-cell receptor domain polypeptide encoded by said nucleic acid.

The modification of the at least one loop region of a T-cell receptor domain polypeptide may result in a substitution and/or insertion of two or more amino acids, preferably point mutations, change of amino acids of whole loops, more preferred the change of at least 2, 3, 4, 5, 6, 7, 8, 9, 10, up to 30 amino acids.

According to the invention the method to introduce modifications is site directed random mutation. With this method two or more specific amino acid residues of the loops are exchanged or introduced using randomly generated inserts into such structural loops. Alternatively preferred is the use of combinatorial approaches.

In another preferred embodiment at least two, in another preferred embodiment at least three structural loop regions of a T-cell receptor domain polypeptide are mutated or modified by random, semi-random or, in particular, by site-directed random mutagenesis methods.

These methods may be used to make amino acid modifications at desired positions of the T-cell receptor domain polypeptide . In these cases positions are chosen randomly, or amino acid changes are made using certain rules. For example certain residues may be mutated to any amino acid, whereas other residues may be mutated to a restricted set of amino acids. This can be achieved in a stepwise fashion by alternating of cycles of mutation and selection or simultaneously.

A preferred method according to the invention refers to a randomly modified nucleic acid molecule coding for a T-cell receptor domain polypeptide which comprises at least one nucleotide repeating unit within a structural loop coding region having the sequence 5'-NNS-3', 5 -NNN-3 or 5 - NNK-3. In some embodiments the modified nucleic acid comprises nucleotide codons selected from the group of TMT, WMT, RMC, RMG, MRT, SRC, KMT, RST, YMT, MKC, RSA, RRC, NNK, NNN, NNS or any combination thereof (the coding is according to IUPAC).

The randomly modified nucleic acid molecule may comprise the above identified repeating units, which code for all known naturally occurring amino acids or a subset thereof.

The modification of the nucleic acid molecule may be performed by introducing synthetic oligonucleotides into a larger segment of nucleic acids or by de novo synthesis of a complete nucleic acid molecule. Synthesis of nucleic acid may also be performed with tri-nucleotide building blocks which would reduce the number of nonsense sequence combinations if a subset of amino acids is to be encoded (e.g. Yanez et al. Nucleic Acids Res. (2004) 32:e158; Virnekas et al. Nucleic Acids Res. (1994) 22:5600-5607).

Preferably the positions to be modified are surface exposed amino acids. Surface exposition of amino acids of structural loops can be judged from known protein structures of T-cell receptor domain polypeptides and by analogy (homology) for such amino acid sequences for which no experimentally determined structure is available.

In a preferred embodiment of the invention the modifications introduced into the at least one structural loop comprise at least 2, 3, 4, 5, or 6 amino acids not naturally occurring at the respective site of the structural loop of the non-modified T-cell receptor domain polypeptide.

The modification of amino acids may preferentially be biased in order to introduce into structural loop regions amino acids which are known to be frequently involved in protein-protein interactions (e.g. Lea & Stewart (1995) FASEB J. 9:87-93; Fellhouse et al. (2006) J. Mol. Biol. 357:100-114; Adib-Conquuy et al. (1998) International Immunology 10:341-346; Lo Conte et al. (1999) J. Mol. Biol. 285:2177-2198; Zemlin et al. (2003) J. Mol. Biol. 334:733-749).

In one preferred embodiment, a library of polypeptide variants comprising T-cell receptor domain polypeptides of the invention is used as a pool for selection wherein the modifications contain or introduce at least one, more preferably at least two amino acids per modified structural loop, preferably out of the group of amino acids tryptophane, tyrosine, phenylalanine, histidine, isoleucine, serine, methionine, alanine and asparagine.

The modification of the T-cell receptor domain polypeptide structural loop according to the present invention is preferably a deletion, substitution or an insertion of one or more amino acids.

According to the present invention at least 2, preferably at least 3, 4, 5, 6, 7, 8, 9, 10 and up to 30 amino acids are deleted, substituted with other amino acids (also with modified amino acids) or inserted into the structural loop region of the T-cell receptor domain polypeptide. However, the maximum number of amino acids inserted into a structural loop region of a T-cell receptor domain polypeptide may not exceed the number of 30, preferably 25, more preferably 20 amino acids.

As is well known in the art, there are a variety of selection technologies that may be used for the identification and isolation of proteins with certain binding characteristics and affinities, including, for example, display technologies such as phage display, ribosome display, cell surface display, and the like, as described below. Methods for production and screening of TCR variants are well known in the art.

The nucleic acid molecules encoding the modified T-cell receptor domain polypeptides (and always included throughout the whole specification below: T-cell receptors and T-cell receptor fragments comprising a modified T-cell receptor domain polypeptide) may be cloned into host cells, expressed and assayed for their binding specificities. These practices are carried out using well-known procedures, and a variety of methods that may find use in the present invention are described in Molecular Cloning--A Laboratory Manual, 3.sup.rd Ed. (Maniatis, Cold Spring Harbor Laboratory Press, New York, 2001), and Current Protocols in Molecular Biology (John Wiley & Sons). The nucleic acids that encode the modified T-cell receptor domain polypeptides may be incorporated into an expression vector in order to express said T-cell receptor domain polypeptides. Expression vectors typically comprise a T-cell receptor domain polypeptide operably linked - that is placed in a functional relationship - with control or regulatory sequences, selectable markers, any fusion partners, and/or additional elements. The modified T-cell receptor domain polypeptide may be produced by culturing a host cell transformed with nucleic acid, preferably an expression vector, containing nucleic acid encoding the modified T-cell receptor domain polypeptide, under the appropriate conditions to induce or cause expression of the modified T-cell receptor domain polypeptide. The methods of introducing exogenous nucleic acid molecules into a host are well known in the art, and will vary with the host used. Of course, also non-cellular or cell-free expression systems for the expression of modified T-cell receptor domain polypeptides may be employed.

The modified T-cell receptor domain polypeptides can be purified or isolated after expression. Modified T-cell receptor domain polypeptides may be isolated or purified in a variety of ways known to those skilled in the art. Standard purification methods include chromatographic techniques, electrophoretic, immunological, precipitation, dialysis, filtration, concentration, and chromatofocusing techniques. Purification can often be enabled by a particular fusion partner. For example, TCRs may be purified using glutathione resin if a GST fusion is employed, Ni²⁺-affinity chromatography if a His-tag is employed or immobilized anti-flag antibody if a flag-tag is used. For general guidance in suitable purification techniques, see e.g. Scopes, "Protein Purification: Principles and Practice", 1994, 3rd ed., Springer-Science and Business Media Inc., NY or Roe, "Protein Purification Techniques : A Practical Approach", 2001, Oxford University Press. Of course, it is also possible to express the modified T-cell receptor domain polypeptide on the surface of a host, in particular on the surface of a bacterial, insect or yeast cell or on the surface of phages or viruses.

Modified T-cell receptor domain polypeptides may be screened using a variety of methods, including but not limited to those that use in vitro assays, in vivo and cell-based assays, and selection technologies. Automation and high-throughput screening technologies may be utilized in the screening procedures. Screening may employ the use of a fusion partner or label, for example an enzyme, an immune label, isotopic label, or small molecule label such as a fluorescent or colorimetric dye or a luminogenic molecule.

In a preferred embodiment, the functional and/or biophysical properties of the T-cell receptor domain polypeptides are screened in an in vitro assay. In a preferred embodiment, the TCR is screened for functionality, for example its ability to catalyze a reaction or its binding specificity, cross reactivity and/or affinity to its target.

In another preferred embodiment, the favourable modified T-cell receptor domain polypeptides may be selected in vivo, e.g. by introducing it into a cell or an organism. The specifically binding variants may be isolated either from body fluid such as blood or lymphatic liquid or from specific organs, depending on the required properties of the modified domains.

Assays may employ a variety of detection methods including but not limited to chromogenic, fluorescent, luminescent, or isotopic labels.

As is known in the art, some screening methods select for favourable members of a library. The methods are herein referred to as "selection methods", and these methods find use in the present invention for screening modified T-cell receptor domain polypeptides. When variant T-cell receptor domain polypeptide libraries are screened using a selection method, only those members of a library that are favourable, that is which meet some selection criteria, are propagated, isolated, and/or observed. As will be appreciated, because only the fittest variants are observed, such methods enable the screening of libraries that are larger than those screenable by methods that assay the fitness of library members individually. Selection is enabled by any method, technique, or fusion partner that links, covalently or non-covalently, the phenotype of T-cell receptor domain polypeptide with its genotype, that is the function of a T-cell receptor domain polypeptide with the nucleic acid that encodes it. For example the use of phage display as a selection method is enabled by the fusion of library members to a phage coat protein. Most frequently used is the filamentous phage gene III protein, however also other coat proteins such as protein VIII, protein VII, protein VI and protein IX can be used. In this way, selection or isolation of modified T-cell receptor domain polypeptides that meet some criteria, for example binding affinity to the T-cell receptor domain polypeptide's target, also selects for or isolates the nucleic acid that encodes it. Once isolated, the gene or genes encoding modified T-cell receptor domain polypeptides may then be amplified. This process of isolation and amplification, referred to as panning, may be repeated, allowing favourable T-cell receptor domain polypeptide variants in the library to be enriched. Nucleic acid sequencing of the attached nucleic acid ultimately allows for gene identification.

A variety of selection methods are known in the art that may find use in the present invention for screening T-cell receptor domain polypeptide libraries (WO04044004A2, WO05116646A1 and WO9839482A1; Dunn et al. (2006) Protein Sci. 15:710-721; Richmann et al. (2006) Protein Eng Des Sel. 19:255-264). These include but are not limited to all the techniques that have been used for selection of specific antibodies and peptides such as phage display (Phage display of peptides and antibodies: a laboratory manual, Kay et al., 1996, Academic Press, San Diego, Calif., 1996; Lowman et al., 1991, Biochemistry 30:10832-10838; Smith, 1985, Science 228:1315-1317) and its derivatives such as selective phage infection (Malmborg et al., 1997, J Mol Biol 273:544-551), selectively infective phage (Krebber et al., 1997, J Mol Biol 268:619-630), and delayed infectivity panning (Benhar et al., 2000, J Mol Biol 301:893-904), cell surface display (Witrrup, 2001, Curr Opin Biotechnol, 12:395-399) such as display on bacteria (Georgiou et al., 1997, Nat Biotechnol 15:29-34; Georgiou et al., 1993, Trends Biotechnol 11:6-10; Lee et al., 2000, Nat Biotechnol 18:645-648; Jun et al., 1998, Nat Biotechnol 16:576-80), yeast (Boder & Wittrup, 2000, Methods Enzymol 328:430-44; Boder & Wittrup, 1997, Nat Biotechnol 15:553-557), and mammalian cells (Whitehorn et al., 1995, Bio/technology 13:1215-1219), as well as in vitro display technologies (Amstutz et al., 2001, Curr Opin Biotechnol 12:400-405) such as polysome display (Mattheakis et al., 1994, Proc Natl Acad Sci USA 91:9022-9026), ribosome display (Hanes et al., 1997, Proc Natl Acad Sci USA 94:4937-4942), mRNA display (Roberts & Szostak, 1997, Proc Natl Acad Sci USA 94:12297-12302; Nemoto et al., 1997, FEBS Lett 414:405-408), and ribosome-inactivation display system (Zhou et al., 2002, J Am Chem Soc 124, 538-543).

Other selection methods that may find use in the present invention include methods that do not rely on display, such as in vivo methods including but not limited to periplasmic expression and cytometric screening (Chen et al., 2001, Nat Biotechnol 19:537-542), the fragment complementation assay (Johnsson & Varshavsky, 1994, Proc Natl Acad Sci USA 91:10340-10344; Pelletier et al., 1998, Proc Natl Acad Sci USA 95:12141-12146), and the yeast two hybrid screen (Fields & Song, 1989, Nature 340:245-246) used in selection mode (Visintin et al., 1999, Proc Natl Acad Sci USA 96:11723-11728). In an alternate embodiment, selection is enabled by a fusion partner that binds to a specific sequence on the expression vector, thus linking covalently or noncovalently the fusion partner and associated T-cell receptor domain polypeptide library member with the nucleic acid that encodes them. For example, WO9308278 describe such a fusion partner and technique that may find use in the present invention. In an alternative embodiment, in vivo selection can occur if expression of the T-cell receptor domain polypeptide imparts some growth, reproduction, or survival advantage to the cell.

Some selection methods are referred to as "directed evolution" methods. Those methods include the mating or breeding of favourable sequences during selection, sometimes with the incorporation of new mutations. As will be appreciated by those skilled in the art, directed evolution methods can facilitate identification of the most favourable sequences in a plurality of polypeptides, and can increase the diversity of sequences that are screened. A variety of directed evolution methods are known in the art that may find use in the present invention for generating and screening T-cell receptor domain polypeptides.
variants, including but not limited to DNA shuffling (PCT WO00/42561; PCT WO 01/70947), exon shuffling (Kolkman & Stemmer, 2001, Nat Biotechnol 19:423-428), family shuffling (Crameri et al., 1998, Nature 391:288-291), selective combinatorial randomization (WO03012100, WO04018674A1), Random Chimeragenesis on Transient Templates (Coco et al., 2001, Nat Biotechnol 19:354-359), molecular evolution by staggered extension process (StEP) in vitro recombination (Zhao et al., 1998, Nat Biotechnol 16:258-261; Shao et al., 1998, Nucleic Acids Res 26:681-683), exonuclease mediated gene assembly (U.S. Pat. No. 6,352,842; U.S. Pat. No. 6,361,974), Gene Site Saturation Mutagenesis (U.S. Pat. No. 6,358,709), Gene Reassembly (U.S. Pat. No. 6,358,709), SCRATCHY (Lutz et al., 2001, Proc Natl Acad Sci USA 98:11248-11253), DNA fragmentation methods (Kikuchi et al., Gene 236:159-167), single-stranded DNA shuffling (Kikuchi et al., 2000, Gene 243:133-137), and directed evolution antibody engineering technology (Applied Molecular Evolution) (U.S. Pat. No. 5,824,514; U.S. Pat. No. 5,817,483; U.S. Pat. No. 5,814,476; U.S. Pat. No. 5,763,192; U.S. Pat. No. 5,723,323).
In a preferred embodiment, T-cell receptor domain polypeptide variants are screened using one or more cell-based or in vivo assays. For such assays, purified or non-purified modified T-cell receptor domain polypeptides are typically added exogenously such that cells are exposed to individual modified T-cell receptor domain polypeptides or pools of modified T-cell receptor domain polypeptides belonging to a library. These assays are typically, but not always, based on the desired function of the T-cell receptor domain polypeptide; that is, the ability of the T-cell receptor domain polypeptide modified according to the invention to bind to its target and to mediate some biochemical event, for example effector function, serum half life, ligand/receptor binding inhibition, apoptosis, and the like. Such assays often involve monitoring the response of cells to the T-cell receptor domain polypeptide, for example cell survival, cell death, change in cellular morphology, or transcriptional activation such as cellular expression of a natural gene or reporter gene. For example, such assays may measure the ability of T-cell receptor domain polypeptide variants to elicit ADCC, ADCP or CDC. For some assays additional cells or components, that is in addition to the target cells, may need to be added, for example serum complement, or effector cells such as peripheral blood monocytes (PBMCs), NK cells, macrophages, and the like. Such additional cells may be from any organism, preferably humans, mice, rat, rabbit, and monkey. T-cell receptor domain polypeptides may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. For example, caspase staining assays may enable to measure apoptosis, and uptake or release of radioactive substrates or fluorescent dyes may enable cell growth or activation to be monitored. Alternatively, dead or damaged target cells may be monitored by measuring the release of one or more natural intracellular components, e.g. lactate dehydrogenase. Transcriptional activation may also serve as a method for assaying function in cell-based assays. In this case, response may be monitored by assaying for natural genes which may be upregulated, for example the release of certain interleukins may be measured, or alternatively readout may be via a reporter system. Cell-based assays may also involve the measure of morphological changes of cells as a response to the presence of modified T-cell receptor domain polypeptides. Cell types for such assays may be prokaryotic or eukaryotic, and a variety of cell lines that are known in the art may be employed.
Alternatively, cell-based screens may be performed using cells that have been transformed or transfected with nucleic acids encoding the variant T-cell receptor domain polypeptides. In this case, T-cell receptor domain polypeptide variants of the invention are not added exogenously to the cells (e.g. analogue to Auf der Maur, 2004, Methods, 34:215-224). In another alternative method, the cell-based screen utilizes cell surface display. A fusion partner can be employed that enables display of modified T-cell receptor domain polypeptides on the surface of cells (as has been shown for antibody fragments: Witrrup, 2001, Curr Opin Biotechnol, 12:395-399).

In a preferred embodiment, the immunogenicity of the modified T-cell receptor domain polypeptide may be determined experimentally using one or more immunological or cell based assays (e.g. Koren et al., 2002, Current Pharmaceutical Biotechnology 3:349-360; Chirino et al., 2004, Drug Discovery Today 9:82-90; Tangri et al., 2005, J. Immunol. 174:3187-3196; Hermeling et al., 2004, Pharm. Res. 21:897-903). In a preferred embodiment, ex vivo T-cell activation assays are used to experimentally quantitate immunogenicity. In this method, antigen-presenting cells and naive T-cells from matched donors are challenged with a peptide or whole T-cell receptor domain polypeptide of interest one or more times. T-cell activation can be detected using a number of methods, e.g. by monitoring of cytokine release or measuring uptake of tritiated thymidine. In preferred embodiments, LUMINEX technology is used to measure cytokine release (e.g. de Jager et al., Clin. Diagn. Lab. Immunol., 2003, 10:133-139) or interferon gamma production is monitored using Elispot assays (Schmittel et. al., 2000, J. Immunol. Meth., 24: 17-24).

The biological properties of the modified T-cell receptor domain polypeptides may be characterized in cell, tissue, and whole organism experiments. As is known in the art, drugs are often tested in animals, including but not limited to mice, rats, rabbits, dogs, cats, pigs, and monkeys, in order to measure a drug's efficacy for treatment against a disease or disease model, or to measure a drug's pharmacokinetics, toxicity, and other properties. The animals may be referred to as disease models. Therapeutics are often tested in mice, including but not limited to nude mice, SCID mice, xenograft mice, and transgenic mice (including genetic knock-in and knock-out mutants). Such experimentation may provide meaningful data for determination of the potential of the polypeptide variant to be used as a therapeutic. Any organism, preferably mammals, may be used for testing. Because of their genetic similarity to humans, monkeys can be suitable therapeutic models, and thus may be used to test the efficacy, toxicity, pharmacokinetics, or other property of the modified T-cell receptor domain polypeptides. Tests in humans are most frequently required for approval as therapeutics, and thus of course these experiments are contemplated. Thus the modified T-cell receptor domain polypeptide may be tested in humans to determine their therapeutic efficacy, toxicity, immunogenicity, pharmacokinetics, and/or other clinical properties.

The modified T-cell receptor domain polypeptide of the present invention may find use in a wide range of products. In one embodiment the T-cell receptor domain polypeptide variant is used for therapy or prophylaxis, for preparative or analytic use, as a diagnostic, as an industrial compound or a research reagent, preferably as a therapeutic. The T-cell receptor domain polypeptide variant may find use in a T-cell receptor domain polypeptide composition that is monoclonal, oligoclonal or polyclonal. In The modified T-cell receptor domain polypeptides may be used to kill target cells that bear the target antigen, for example cancer cells.
The modified T-cell receptor domain polypeptides may be used to block, antagonize, or agonize the target antigen, for example by antagonizing a cytokine or cytokine receptor. The modified T-cell receptor domain polypeptides may be used to block, antagonize, or agonize the target antigen and kill the target cells that bear the target antigen.
The modified T-cell receptor domain polypeptides may be used to block, antagonize, or agonize growth factors or growth factor receptors and kill the target cells that bear or need the target antigen.
The modified T-cell receptor domain polypeptides may be used to block, antagonize, or agonize enzymes and substrate of enzymes.
The modified T-cell receptor domain polypeptides may be used to neutralize infectious agents such as viruses, bacteria or fungi.
The modified T-cell receptor domain polypeptides may show increased serum half life.
The modified T-cell receptor domain polypeptides may show effector function capabilities.

The modified T-cell receptor domain polypeptides may be used for various therapeutic purposes. For example, a T-cell receptor comprising the modified T-cell receptor domain polypeptide may be administered to a patient to treat a specific disorder. A "patient" includes both, humans and other animals, preferably mammals and most preferably humans. By "specific disorder" herein is meant a disorder that may be ameliorated by the administration of a pharmaceutical composition comprising a modified T-cell receptor domain polypeptide.

A modified T-cell receptor domain polypeptide may be the only therapeutically active agent administered to a patient. Alternatively, the modified T-cell receptor domain polypeptide may be administered in combination with one or more other therapeutic agents, including but not limited to cytotoxic agents, chemotherapeutic agents, cytokines, growth inhibitory agents, anti-hormonal agents, kinase inhibitors, anti-angiogenic agents, cardioprotectants, or other therapeutic agents. The modified T-cell receptor domain polypeptide may be administered concomitantly with one or more other therapeutic regimens. For example, a T-cell receptor variant may be administered to the patient along with chemotherapy, radiation therapy, or both chemotherapy and radiation therapy. The modified T-cell receptor domain polypeptide may be administered in conjunction with one or more antibodies. Alternatively, the modified T-cell receptor domain polypeptide and one or more other anti-cancer therapies are employed to treat cancer cells ex vivo. It is contemplated that such ex vivo treatment may be useful in bone marrow transplantation and particularly, autologous bone marrow transplantation. It is of course contemplated that the T-cell receptor domain polypeptide of the invention can be employed in combination with still other therapeutic techniques such as surgery.

A variety of other therapeutic agents may find use for administration with the modified T-cell receptor domain polypeptide. The modified T-cell receptor domain polypeptide may be administered with an anti-angiogenic agent, which is a compound that blocks, or interferes to some degree with the development of blood vessels. The anti-angiogenic factor may, for instance, be a small molecule or a protein, for example an antibody, Fc fusion, or cytokine, that binds to a growth factor or growth factor receptor involved in promoting angiogenesis. The preferred anti-angiogenic factor herein is an antibody that binds to Vascular Endothelial Growth Factor (VEGF). Alternatively, the modified T-cell receptor domain polypeptide may be administered with a therapeutic agent that induces or enhances adaptive immune response, for example an antibody that targets CTLA-4. Alternatively, the modified T-cell receptor domain polypeptide may be administered with a tyrosine kinase inhibitor, which is a molecule that inhibits to some extent tyrosine kinase activity of a tyrosine kinase. Alternatively, the modified T-cell receptor domain polypeptides may be administered with a cytokine. By "cytokine" as used herein is meant a generic term for proteins released by one cell population that act on another cell as intercellular mediators including chemokines.

Pharmaceutical compositions are contemplated wherein modified T-cell receptor domain polypeptides of the same or different specificities and one or more therapeutically active agents are formulated. Formulations of the polypeptide variants are prepared for storage by mixing said modified T-cell receptor domain polypeptides having the desired degree of purity with optional pharmaceutically acceptable carriers, excipients or stabilizers (Remington's Pharmaceutical Sciences, 1980, 16th edition, Osol, A. Ed.,), in the form of lyophilized formulations or aqueous solutions. The formulations to be used for in vivo administration are preferably sterile. This is readily accomplished by filtration through sterile filtration membranes or other methods. The modified T-cell receptor domain polypeptide and other therapeutically active agents disclosed herein may also be formulated as immunoliposomes, and/or entrapped in microcapsules.

Administration of the pharmaceutical composition comprising a modified T-cell receptor domain polypeptide or a mixture of different modified T-cell receptor domain polypeptides, preferably in the form of a sterile aqueous solution, may be performed in a variety of ways, including, but not limited to, orally, subcutaneously, intravenously, intranasally, intraotically, transdermally, topically (e.g., gels, salves, lotions, creams, etc.), intraperitoneally, intramuscularly, intrapulmonary, vaginally, parenterally, rectally, or intraocularly.

According to a preferred embodiment of the present invention the expression system comprises a vector. Any expression vector known in the art may be used for this purpose as appropriate.

The modified T-cell receptor domain polypeptide is preferably expressed in a host, preferably in a bacterium, in yeast, in a plant cell, in an insect cell, in an animal cell or mammalian cell or in an organ of a plant or animal or in a complete plant or animal.

A wide variety of appropriate host cells may be used to express the modified polypeptide of the invention, including but not limited to mammalian cells (animal cells) plant cells, bacteria (e.g. Bacillus subtilis, Escherichia coli), insect cells, and yeast (e.g. Pichia pastoris, Saccharomyces cerevisiae). For example, a variety of cell lines that may find use in the present invention are described in the ATCC cell line catalog, available from the American Type Culture Collection. Furthermore, also plants and animals may be used as hosts for the expression of the T-cell receptor domain polypeptide according to the present invention. The expression as well as the transfection vectors or cassettes may be selected according to the host used.

Of course also non-cellular or cell-free protein expression systems may be used. In vitro transcription/translation protein expression platforms, that produce sufficient amounts of protein offer many advantages of a cell-free protein expression, eliminating the need for laborious up- and down-stream steps (e.g. host cell transformation, culturing, or lysis) typically associated with cell-based expression systems.
The present disclosure relates to a method for manufacturing a T-cell receptor domain polypeptide, the method comprising at least one modification in a structural loop of said T-cell receptor domain polypeptide and determining the binding of said molecule to an epitope of an antigen, wherein the unmodified molecule does not significantly bind to said epitope, comprising the steps of:
- providing a nucleic acid encoding a T-cell receptor domain polypeptide comprising at least one structural loop region,
- modifying at least one nucleotide residue in said at least one structural loop region,
- transferring said modified nucleic acid in an expression system,
- expressing said modified T-cell receptor domain polypeptide,
- contacting the expressed modified T-cell receptor domain polypeptide with an epitope,
- determining whether said modified T-cell receptor domain polypeptide binds to said epitope, and
- providing the modified T-cell receptor domain polypeptide binding to said epitope and optionally finishing it to a pharmaceutical preparation.

Described herein is a method for manufacturing a multi-specific molecule binding specifically to at least one first molecule or a pharmaceutical preparation thereof comprising at least one modification in each of at least one structural loop region of said T-cell receptor domain polypeptide and determining the specific binding of said at least one loop region to at least one second molecule selected from the group consisting of allergens, tumor associated antigens, self antigens, enzymes, Fc-receptors, proteins of the complement system, serum molecules, bacterial antigens, fungal antigens, protozoal antigens and viral antigens, wherein the T-cell receptor domain polypeptide containing an unmodified structural loop region does not specifically bind to said at least one second molecule, comprising the steps of:
- providing a nucleic acid encoding a T-cell receptor domain polypeptide binding specifically to at least one first molecule comprising at least one structural loop region,
- modifying at least one nucleotide residue of at least one of said loop regions encoded by said nucleic acid,
- transferring said modified nucleic acid in an expression system,
- expressing said modified T-cell receptor domain polypeptide,
- contacting the expressed modified T-cell receptor domain polypeptide with said at least one second molecule, and
- determining whether said modified T-cell receptor domain polypeptide binds specifically to the second molecule and
- providing the modified T-cell receptor domain polypeptide binding specifically to said at least one second molecule and optionally finishing it to a pharmaceutical preparation.

A TCR may consist of an alpha (or a gamma) chain and a beta (or a delta) chain, which form together a variable region binding to a specific binding partner, and the second specificity may be formed by modified structural loops of either the alpha (or gamma) chain or the beta (or delta) chain variable or constant domain. The binding site may also be formed by at least one or more than one non-CDR loops on two variable or constant domains (e.g. a heavy chain variable domain and a light chain variable domain which may be structurally neighboured).
The modified T-cell receptor or derivative may be a complete T-cell receptor or a T-cell receptor fragment (e.g. soluble TCR, single-chain-TCR) comprising at least one modified T-cell receptor domain polypeptide.

It may bind mono- or multi-valently to binding partners or even with different valency for the different binding partners, depending on the design. For example, a T-cell receptor fragment or, equivalently an scTCR may be engineered in such a way that the structural loops of both variable domains are separately engineered to bind to the same epitope as the binding site formed by the CDRs, resulting in a trivalent T-cell receptor or scTCR respectively. If for example the natural binding site formed by the CDRs recognizes a different target epitope than the engineered variable domains then the resulting TCR fragment or scTCR will bind monovalently to the first target, and bivalently to the second target which is bound independantly by the modified structural loops of the variable domains respectively. This modular design principle can be applied in numerous different ways as will be obvious to those skilled in the art.

As there are a number of various structural loops available for selection and design of a specific binding site in the non-CDR regions of alpha (gamma) and beta (delta) domains it is possible to design T-cell receptor derivatives with even more than two specificities. For example, Valpha and Vbeta domains recognizing a first target by their CDRs can be engineered separately to bind specifically to different (second and third) targets through interactions mediated by the modified structural loops in the respective variable domain. Thus, a trispecific T-cell receptor or a fragment therof such as a single chain T-cell receptor, which binds monovalenty to each of its various targets can be generated.

The specific binding domains within one polypeptide chain may be connected with or without a peptide linker and may not necessarily be in the natural order.
Neither constant nor variable domains of T-cell receptors mediate effector functions which is the reason why T-cell receptor fragments do not show ADCC, ADCP or CDC. With the current invention it is possible to design a T-cell receptor binding to effector molecules such as Fc receptors and complement proteins. Modified loops in T-cell receptor domains can be selected from a library of modified loop structures or designed to bind to one or more effector molecules. A T-cell receptor or a fragment thereof with such effector molecule binding sites would enable effector functions similar to antibodies and could be designed depending on the requirements to show strong or weak ADCC and ADCP and/or complement activation.

In order to select for potential effector function of such T-cell receptor domain polypeptides, libraries comprising mutant T-cell receptor domain polypeptides may be selected for binding to Fc-receptors and/or complement factors such as C1q. Fcgamma receptors for selection may be provided either on the surface of cells expressing naturally the respective receptors or by expression and purification of the extracellular part of the respective receptor. IFN-g stimulated U937 cells (CRL-1503, American Type Culture Collection) can be used as target cells for the isolation of phage displayed modified T-cell receptor domain polypeptides that bind specifically to the high- affinity IgG receptor, FcgammaRI (similar to Berntzen et al., 2006, Protein Eng Des Sel. 19(3):121-8). Binding to the Fc receptor can be tested for by FACS using U937 cells as target which are stained specifically with selected modified T-cell receptor domain polypeptides. Furthermore, the extracellular domains of human Fcgamma receptors can be cloned and expressed as soluble proteins or fusion proteins and used for analysis of the specific binding of potential binding partners (e.g. as in Berntzen et al., 2005, J Immunol Methods. 298(1-2):93-104). The identification and characterisation of modified T-cell receptor domain polypeptides specifically binding to complement factor C1q can be performed essentially similarily (e.g. as in Lauvrak et al. 1997 Biol Chem. 378(12):1509-19).

In order to increase in vivo half life of a molecule comprising such a T-cell receptor domain polypeptide binding to FcRn may be selected for with libraries of mutant T-cell receptor domain polypeptides.

FcRn-receptors for selection may be provided either on the surface of cells expressing naturally the respective receptors or by expression and purification of the extracellular part of the respective receptor. A first screening on FcRn may select for mutant T-cell receptor domain polypeptides (or molecules comprising such mutant T-cell receptor domain polypeptides) which can further be tested in vitro and even further characterized in FACS experiments by binding to cells expressing FcRn receptor. Screening and selection may also consider pH dependencies in binding to FcRn (as described in PCT WO02/060919; PCT WO97/34631 ). It can be further characterized by affinity ranking of binding to various recombinant FcRn, isoforms and allotypes e.g with surface plasmon resonance techniques (e.g. as in Dall' Acqua et al. Journal of Immunology, 2002, 169: 5171 5180).

The T-cell receptors comprise preferably either an alpha and beta chain or a gamma and delta chain of the T-cell receptor or a part thereof.

The modified T-cell receptor may comprise an alpha or beta chain or a gamma and delta chain, at least one variable domain.

The T-cell receptor comprises preferably at least one constant and/or at least one variable domain of a T-cell receptor or a part thereof.

Another preferred T-cell receptor consists of a domain of an alpha, beta, gamma or delta chain, or a part thereof, with at least two structural loop regions, and is characterised in that said at least two structural loop regions comprise at least two amino acid modifications forming at least two modified structural loop regions, wherein said at least two modified structural loop regions bind specifically to at least one epitope of an antigen.

According to a preferred embodiment of the present invention the specific binding of the modified T-cell receptor domain polypeptide to a molecule is determined by a binding assay selected from the group consisting of immunological assays, preferably enzyme linked immunosorbent assays (ELISA), surface plasmon resonance assays, saturation transfer difference nuclear magnetic resonance spectroscopy, transfer NOE (trNOE) nuclear magnetic resonance spectroscopy, competitive assays, tissue binding assays, live cell binding assays and cellular extract assays.

Binding assays can be carried out using a variety of methods known in the art, including but not limited to FRET (Fluorescence Resonance Energy Transfer) and BRET (Bioluminescence Resonance Energy Transfer)-based assays, Amplified Luminescent Proximity Homogeneous Assay, Scintillation Proximity Assay, ELISA (Enzyme-Linked Immunosorbent Assay), SPR (Surface Plasmon Resonance), isothermal titration calorimetry, differential scanning calorimetry, gel electrophoresis, and chromatography including gel filtration.

The modified polypeptide is preferably conjugated to a label selected from the group consisting of organic molecules, enzyme labels, radioactive labels, colored labels, fluorescent labels, chromogenic labels, luminescent labels, haptens, digoxigenin, biotin, metal complexes, metals, colloidal gold and mixtures thereof.

The modified T-cell receptor domain polypeptide may be conjugated to other molecules which allow the simple detection of said conjugate in, for instance, binding assays (e.g. ELISA) and binding studies.

Also described herein is a polypeptide comprising a domain of a T-cell receptor or combinations thereof, with at least two structural loop regions, characterised in that said at least two structural loop regions each comprise at least one amino acid modification forming at least two modified structural loop regions, wherein said at least two modified structural loop regions bind specifically to at least one epitope of an antigen.

It is preferred to combine molecularly at least one modified T-cell receptor domain polypeptide (= binding to the specific partner via the non-variable sequences or structural loops) with at least one other binding molecule which can be an antibody, antibody fragment, a soluble receptor, a ligand or another modified T-cell receptor domain polypeptide.

The other binding molecule combined with the at least one modified T-cell receptor domain polypeptide is selected from the group consisting of proteinaceous molecules, nucleic acids, and carbohydrates.

The structural loop regions of the modified T-cell receptor domain polypeptides may specifically bind to any kind of binding molecules, in particular to proteinaceous molecules, proteins, peptides, polypeptides, nucleic acids, glycans, carbohydrates, lipids, small and large organic molecules, inorganic molecules. Of course, the modified T-cell receptor domain polypeptide may comprise at least two loop regions whereby each of the loop regions may specifically bind to different molecules or epitopes.

According to a preferred embodiment of the present disclosure the molecule binding to the modified structural loop region is selected from the group consisting of tumor associated antigens, in particular EpCAM, tumor-associated glycoprotein-72 (TAG-72), tumor-associated antigen CA 125, Prostate specific membrane antigen (PSMA), High molecular weight melanoma-associated antigen (HMW-MAA), tumor-associated antigen expressing Lewis Y related carbohydrate, Carcinoembryonic antigen (CEA), CEACAM5, HMFG PEM, mucin MUC1, MUC18 and cytokeratin tumor-associated antigen, bacterial antigens, viral antigens, allergens, allergy related molecules IgE, cKIT and Fc-epsilon-receptorI, IRp60, IL-5 receptor, CCR3, red blood cell receptor (CR1), human serum albumin, mouse serum albumin, rat serum albumin, neonatal Fc-gamma-receptor FcRn, Fc-gamma-receptors Fc-gamma RI, Fc-gamma-RII, Fc-gamma RIII, Fc-alpha-receptors, Fc-epsilon-receptors, fluorescein, lysozyme, toll-like receptor 9, erythropoietin, CD2, CD3, CD3E, CD4, CD10, CD11, CD11a, CD14, CD16, CD18, CD19, CD20, CD22, CD23, CD25, CD28, CD29, CD30, CD32, CD33 (p67 protein), CD38, CD40, CD40L, CD52, CD54, CD56, CD64, CD80, CD147, GD3, IL-1, IL-1R, IL-2, IL-2R, IL-4, IL-5, IL-6, IL-6R, IL-8, IL-12, IL-15, IL-18, IL-23, interferon alpha, interferon beta, interferon gamma; FGF20, TNF-alpha, TNFbeta2, TNFalpha, TNFalphabeta, TNF-R1, TNF-RII, FasL, CD27L, CD30L, 4-1BBL, TRAIL, RANKL, TWEAK, APRIL, BAFF, LIGHT, VEG1, OX40L, TRAIL Receptor-1, A1 Adenosine Receptor, Lymphotoxin Beta Receptor, TACI, BAFF-R, EPO; LFA-3, ICAM-1, ICAM-3, integrin beta1, integrin beta2, integrin alpha4/beta7, integrin alpha2, integrin alpha3, integrin alpha4, integrin alpha5, integrin alpha6, integrin alphav, alphaVbeta3 integrin, FGFR-3, Keratinocyte Growth Factor, VLA-1, VLA-4, L-selectin, anti-Id, E-selectin, HLA, HLA-DR, CTLA-4, T cell receptor, B7-1, B7-2, VNRintegrin, TGFbeta1, TGFbeta2, eotaxin1, BLyS (B-lymphocyte Stimulator), complement C5, IgE, IgA, IgD, IgM, IgG, factor VII, CBL, NCA 90, EGFR (ErbB-1), Her2/neu (ErbB-2), Her3 (ErbB-3), Her4 (ErbB4), Tissue Factor, VEGF, VEGFR, endothelin receptor, VLA-4, carbohydrates such as blood group antigens and related carbohydrates, Galili-Glycosylation, Gastrin, Gastrin receptors, tumor associated carbohydrates, Hapten NP-cap or NIP-cap, T cell receptor alpha/beta, E-selectin, P-glycoprotein, MRP3, MRP5, glutathione-S-transferase pi (multi drug resistance proteins), alpha-granule membrane protein(GMP) 140, digoxin, placental alkaline phosphatase (PLAP) and testicular PLAP-like alkaline phosphatase, transferrin receptor, Heparanase I, human cardiac myosin, Glycoprotein IIb/IIIa (GPIIb/IIIa), human cytomegalovirus (HCMV) gH envelope glycoprotein, HIV gp120, HCMV, respiratory syncital virus RSV F, RSVF Fgp, VNRintegrin, Hep B gp120, CMV, gpIIbLIIa, HIV IIIB gp120 V3 loop, respiratory syncytial virus (RSV) Fgp, Herpes simplex virus (HSV) gD glycoprotein, HSV gB glycoprotein, HCMV gB envelope glycoprotein, Clostridium perfringens toxin and fragments thereof.

Preferably, the antigen is selected from the group consisting of pathogen antigen, tumor associated antigen, enzyme, substrate, self antigen, organic molecule or allergen. More preferred antigens are selected from the group consisting of viral antigens, bacterial antigens or antigens from pathogens of eukaryots or phages. Preferred viral antigens include HAV-, HBV-, HCV-, HIV I-, HIV II-, Parvovirus-, Influenza-, HSV-, Hepatitis Viruses, Flaviviruses, Westnile Virus, Ebola Virus, Pox-Virus, Smallpox Virus, Measles Virus, Herpes Virus, Adenovirus, Papilloma Virus, Polyoma Virus, Parvovirus, Rhinovirus, Coxsackie virus, Polio Virus, Echovirus, Japanese Encephalitis virus, Dengue Virus, Tick Borne Encephalitis Virus, Yellow Fever Virus, Coronavirus, respiratory syncytial virus, parainfluenza virus, La Crosse Virus,Lassa Virus,Rabies Viruse, Rotavirus antigens; preferred bacterial antigens include Pseudomonas-, Mycobacterium-, Staphylococcus-, Salmonella-, Meningococcal-, Borellia-, Listeria, Neisseria-, Clostridium-, Escherichia-, Legionella-, Bacillus-, Lactobacillus-, Streptococcus-, Enterococcus-, Corynebacterium-, Nocardia-, Rhodococcus-, Moraxella-, Brucella, Campylobacter-, Cardiobacterium-, Francisella-, Helicobacter-, Haemophilus-, Klebsiella-, Shigella-, Yersinia, Vibrio-, Chlamydia-, Leptospira-, Rickettsia-, Mycobacterium-, Treponema-, Bartonella- antigens. Preferred eukaryotic antigens of pathogenic eukaryotes include antigens from Giardia, Toxoplasma, Cyclospora, Cryptosporidium, Trichinella, Yeasts, Candida, Aspergillus, Cryptococcus, Blastomyces, Histoplasma, Coccidioides.

The modified T-cell receptor domain polypeptide may preferably bind to one of the molecules disclosed above. These molecules comprise also allergens.

The modified polypeptide may be preferably conjugated to a label or reporter molecule selected from the group consisting of organic molecules, enzyme labels, radioactive labels, colored labels, fluorescent labels, chromogenic labels, luminescent labels, haptens, digoxigenin, biotin, metal complexes, metals, colloidal gold and mixtures thereof.

Modified polypeptides conjugated to labels as specified above may be used, for instance, in diagnostic methods.

Also described herein is to the use of a T-cell receptor domain polypeptide obtainable by a method according to the present invention for the preparation of a vaccine for active immunization. Hereby the T-cell receptor domain polypeptide is either used as antigenic drug substance to formulate a vaccine or used for fishing or capturing antigenic structures for use in a vaccine formulation.

Also described herein is the use of a T-cell receptor domain polypeptide obtainable by a method according to the present invention for the preparation of a protein library of molecules comprising modified T-cell receptor domain polypeptides.

Also described herein is a method for specifically binding and/or detecting a target molecule comprising the steps of:
(a) contacting a molecule comprising a modified T-cell receptor domain polypeptide or a molecule comprising a modified T-cell receptor domain 41 polypeptide obtainable by a method according to the present invention with a test sample suspected to contain said target molecule, and
(b) detecting the potential formation of a specific T-cell receptor domain polypeptide/target molecule complex.

Also described herein is a method for specifically isolating a target molecule comprising the steps of:
(a) contacting a molecule comprising a modified T-cell. receptor domain polypeptide or a molecule comprising a modified T-cell receptor domain polypeptide obtainable by a method according to the present invention with a sample containing said target molecule,
(b) separating the specific T-cell receptor domain polypeptide/target molecule complex formed, and
(c) optionally isolating the target molecule from said complex.

The T-cell receptor domain polypeptides may be used to isolate specifically target molecules from a sample. If multi-specific T-cell receptor domain polypeptides are used more than one target molecule may be isolated from a sample. It is especially advantageous using modified T-cell receptor domain polypeptides in such methods because it allows, e.g., to generate a matrix having a homogeneous surface with defined amounts of binding partners (i.e. modified T-cell receptor domain polypeptides) immobilised thereon which are able to bind to the target molecules to be isolated. In contrast thereto, if monospecific binding partners are used no homogeneous matrix can be generated because the single binding partners do not bind with the same efficiency to the matrix.

Also described herein is a method for targeting a compound to a target comprising the steps of:
(a) contacting a molecule comprising a modified T-cell receptor domain polypeptide or a molecule comprising a modified T-cell receptor domain polypeptide obtainable by a method according to the present invention capable to specifically bind to said compound,
(b) delivering the molecule comprising a T-cell receptor domain polypeptide/compound complex to the target.

Modified T-cell receptor domain polypeptides may be used to deliver at least one compound bound to the CDRs and/or modified structural loop regions to a target. Such modified T-cell receptors may be used to target therapeutic substances to a preferred site of action in the course of the treatment of a disease.

Also described herein is a molecule library comprising a T-cell receptor domain polypeptide according to the present invention or obtainable by the method according to the present invention.

Preferred methods for constructing said library can be described herein found above and in the examples. The library described herein may be used to identify T-cell receptor domain polypeptides binding to a distinct molecule.

Described herein is the use of a protein library of polypeptides comprising a T-cell receptor domain polypeptide obtainable by the method according to the present invention for the design of T-cell receptor derivatives.
An existing T-cell receptor can be changed to introduce antigen binding sites into a domain by using a protein library of the respective modified wild-type domain of at least 10, preferably 100, more preferably 1000, more preferably 10000, more preferably 100000, most preferably more than 1000000 variant domains each with at least one modified structural loop. Preferably the variant domains comprise of at least two modified structural loops. The library is then screened for binding to the specific antigen. After molecular characterization for the desired properties the selected domain is cloned into the original T-cell receptor by genetic engineering techniques so that it replaces the wild type region. Alternatively, only the DNA coding for the modified structural loops or coding for the mutated amino acids may be exchanged to obtain a T-cell receptor with the additional binding site for the specific antigen. Alternatively, the modification in the structural loops of the domains may be performed with the domain in its natural context, e.g. in the form of a cell bound T-cell receptor, a soluble T-cell receptor, a ingle-chain TCR a zipper dimerized TCR or a combination thereof with any other molecule. The advantage of this setup is that the screening is performed with modifications in their intended context and therefore any influence of the various modifications on the structure or function of the remainder of the molecule is easily observed.

The choice of the site for the mutated, antigen-specific structural loop is dependent on the structure of the original T-cell receptor and on the purpose of the additional binding site. If, for example, the original T-cell receptor is a single chain TCR modification of structural loops in the two variable domains is possible. If the original T-cell receptor is a soluble TCR with constant and variable domains, structural loops on one side of each variable domain may be modified as well as structural loops on two sides of each constant domain.

To generate a library one may prepare libraries of mutant original molecules which have mutations in one or more structural loops of one or more T-cell receptor domains. The selection with complete mutated original molecules may have some advantages as the selection for antigen binding with a modified structural loop will deliver the sterically advantageous modifications. For example, if the complete molecule is a single chain TCR, it may be advantageous to screen the library of mutated original single-chain TCRs for binding to an antigen, followed by screening the specific binders for binding to the antigen which is recognized by the CDR loops (original specificity). In an alternative selection procedure the original - the first - antigen may be bound to the CDR-loops during the screening for binding to an antigen with the modified structural loops. This simultaneous screening may allow for preferential selection of clones that contain mutations in the structural loops which do not negatively affect the binding of the modified T-cell receptor domain to its original target which it recognizes through its CDR loops.

Described herein is a library of variant T-cell receptor domain polypeptides with at least one variant amino acid position in at least one of the structural loops. The library may comprise TCR domains of the alpha, beta, gamma and delta chain or mixtures and molecular combinations thereof. More preferably the variant T-cell receptor domain polypeptides of the library have at least 2, at least 3, at least 4 variant amino acid position in at least one structural loop.

Also described herein is is a single-chain TCR library with at least one variant amino acid position in at least one of the structural loops of any of the domains of the scTCR.

Also described herein is is a zipper-dimerized TCR library with at least one variant amino acid position in at least one of the structural loops of any of the domains of the leucine-zipper-dimeri ed TCR.

Also described herein is is a TCR library with at least one variant amino acid position in at least one of the structural loops of any of the domains of the TCR. Also

Also described herein is a soluble TCR library with at least one variant amino acid position in at least one of the structural loops of any of the domains of the soluble TCR.
The size requirement (i.e. the number of variant proteins) of a protein library comprising mutated T-cell receptor domain polypeptides or fusion molecules of a mutated T-cell receptor domain polypeptide is dependent on the task. In general, a library to generate an antigen binding site de novo needs to be larger than a library used to further modify an already existing engineered antigen binding site formed by a modified structural loop (e.g. for enhancing affinity or changing fine specificity to the antigen).

Also described herein is a polypeptide library or a nucleic acid library comprising a plurality of polypeptides comprising T-cell receptor domains or at least one structural loop region contained in a minidomain, or nucleic acid molecules encoding the same. The library contains members with different modifications, wherein the plurality is defined by the modifications in the at least one structural loop region. The nucleic acid library preferably includes at least 10 different members (with at least one, more preferably at least two, even more preferably at least three, most preferably at least four potential amino acid modifications) and more preferably includes at least 100, more preferably 1000 or 10000 different members (e.g. designed by randomisation strategies or combinatory techniques). Even more diversified individual member numbers, such as at least 1000000 or at least 10000000 are also preferred.

Also described herein is is the combination of two different T-cell receptor domain polypeptides selected from at least two libraries in order to generate multispecific T-cell receptors. These selected specific T-cell receptor domain polypeptides may be combined with each other and with other molecules, similar to building blocks, to design the optimal arrangement of the domains to get the desired properties such as combinations of specificities and/or valencies.

Furthermore, one or more modified T-cell receptor domain polypeptides may be introduced at various or all the different sites of a protein without destruction of the structure of the protein. By such a "domain shuffling" technique new libraries are created which can again be selected for the desired properties.

The preferred library contains T-cell receptor domain polypeptides according to the invention or derivatives thereof.

Described herein is a binding molecule for an antigen (antigen binding molecule) comprising at least one T-cell receptor domain polypeptide and at least one structural loop region thereof being modified according to the present invention to bind to the antigen, wherein said binding molecule has no relevant and/or specific binding activity with its CDR-loops; however with a newly introduced specific binding activity in the structural loop region.

Also for the antigen binding molecules it is preferred that the new antigen binding sites in the structural loops are introduced by randomising technologies, i.e. by modifying one or more amino acid residues of at least two structural loops by randomisation techniques or by introducing randomly generated inserts into such structural loops. Alternatively preferred is the use of combinatorial approaches.

Also described herein is a modified T-cell receptor having an antigen binding site foreign to the unmodified T-cell receptor and incorporated in one, two, three or more structural loops of at least one domain. The term "foreign" means that the antigen binding site is not naturally formed by the specific structural loop regions of the T-cell receptor domain.

Also described herein is a modified T-cell receptor having an antigen binding site foreign to the unmodified T-cell receptor and incorporated in one, two, three or more structural loops of al least one domain, wherein said modified T-cell receptor binds to said antigen with an affinity of at least 10³ mol⁻¹, at least 10⁴ mol⁻¹, at least 10⁵ mol⁻¹, at least 10⁶ mol⁻¹, at least 10⁷ mol⁻³, at least 10⁸ mol⁻¹, or at least 10⁹ mol⁻¹.

Preferred T-cell receptor domain polypeptides comprise at least two antigen binding sites, the first site binding to a first epitope, and the second site binding to a second epitope. a

Preferably, a T-cell receptor domain polypeptide as described herein comprises at least three loop regions, the first loop region binding to a first epitope, and the second and third loop region binding to a second epitope. Either the at least first or at least second and third loop region or both may contain a structural loop. The T-cell receptor domains include the fragments thereof known in the art to be functional which contain the structural loop regions modified according to the present invention.

Preferably, the T-cell receptor is composed of at least two T-cell receptor domains, or a part thereof including a minidomain, and each domain contains at least one antigen binding site.

Also preferred is a T-cell receptor domain polypeptide which comprises at least one domain of the variable region of a T-cell receptor and at least one domain of the constant region of a T-cell receptor; for example, a variable domain, which is modified in at least two structural loops linked to a constant domain.

The preferred T-cell receptor domain polypeptide comprises a domain that has at least 50% homology with the unmodified domain.

The term "homology" indicates that polypeptides have the same or conserved residues at a corresponding position in their primary, secondary or tertiary structure. The term also extends to two or more nucleotide sequences encoding the homologous polypeptides.

"Homologous TCR domain" means a TCR domain having at least about 50% amino acid sequence identity with regard to a full-length native sequence TCR framework domain sequence or any other fragment of a full-length TCR domain sequence as disclosed herein. Preferably, a homologous TCR domain will have at least about 50% amino acid sequence identity, preferably at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native TCR domain sequence, or any other specifically defined fragment of a full-length TCR domain sequence as disclosed herein.

"Percent (%) amino acid sequence identity" with respect to the TCR domain sequences identified herein is defined as the percentage of amino acid residues in a candidate sequence that are identical with the amino acid residues in the specific TCR domain sequence, after aligning the sequence and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

% amino acid sequence identity values may be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span=1, overlap fraction=0.125, word threshold (T)=11, and scoring matrix=BLOSUM62. When WU-BLAST-2 is employed, a % amino acid sequence identity value is determined by dividing (a) the number of matching identical amino acid residues between the amino acid sequence of the TCR domain of interest having a sequence derived from the native TCR domain and the comparison amino acid sequence of interest (i.e., the sequence against which the TCR domain of interest is being compared which may be the unmodified TCR domain) as determined by WU-BLAST-2 by (b) the total number of amino acid residues of the non-randomized parts of the TCR domain of interest. For example, in the statement "a polypeptide comprising an amino acid sequence X which has or having at least 80% amino acid sequence identity to the amino acid sequence Y", the amino acid sequence A is the comparison amino acid sequence of interest and the amino acid sequence B is the amino acid sequence of the TCR domain of interest.

A polypeptide described herein may be a T-cell receptor or a bispecific single chain T-cell receptor or a bispecific zipper-dimerized T-cell receptor or a bispecific soluble T-cell receptor. Further preferred is that the polypeptide comprises a bispecific domain or a part thereof.

The polypeptide may be used for any purpose known in the art for T-cell receptors and immunoglobulins but also enables applications which are depending on the combination of specificities introduced by the present invention. Accordingly, the polypeptides are preferably used for therapeutic and prophylactic use (e.g. as an active or passive immunotherapy, for immunomodulation); for preparative and analytic use and for diagnostic use.

Also described herein is a kit of binding partners containing
(a) a polypeptide comprising a modified T-cell receptor domain polypeptide having an antigen binding site incorporated in one or more structural loops, and
(b) a binding molecule containing an epitope of said antigen.

Such a binding molecule of this kit according to the present invention may be used for identifying the binding specificity of the polypeptide comprising a modified T-cell receptor domain polypeptide according to the present invention. By using the binding molecule of this kit, the potency of the modified polypeptide may be determined.
Potency as defined here is the binding property of the modified molecule of the invention to its antigen. The binding can be determined quantitatively and/or qualitatively in terms of specificity and/or affinity and/or avidity as used for quality control purposes.

Moreover, the binding molecule of a kit may be used for selecting the polypeptide comprising a modified T-cell receptor domain polypeptide from a library consisting of at least 10, preferably at least 100, more preferably at least 1000, more preferred at least 10000, especially at least 100000 polypeptides with different modifications in the structural loops.

In accordance with the present invention, one of the key features of the present invention is that the engineering of the T-cell receptor domain polypeptides takes place in regions which are not normally involved in antigen binding, in other words, in regions other than the CDRs of a TCR variable domain. It was observed that the specific fold of T-cell receptor domains allows the introduction of random mutations in regions which are structurally analogous to the CDRs but different in position in sequence and structure. The regions identified are, like CDRs, loop regions connecting the beta strands of the immunoglobulin fold of the T-cell receptor domain polypeptide. These structural loop regions can be mutated as described without affecting the binding of the domains of the T-cell receptor that is mediated through the CDR loops. By mutating said structural loop regions, a new molecular binding surface or binding pocket can be generated that is similar in size and shape to the binding surface or binding pocket formed by the CDR loops of the natural antigen binding site of a T-cell receptor. Since the structural loops can also be extended by the insertion of additional amino acids, the architecture of the newly generated binding site can be adjusted to the target to which it should bind. For example, deep binding pockets which are especially suitable for the binding of small molecules can be preferentially formed by long loops, i.e. structural loops with additional amino acids inserted in their sequence, whereas rather flat binding surfaces, which are well suited to bind to targets with a large, flat molecular surface are preferentially formed when the residues in the structural loops are mutated without the insertion of additional residues

More specifically, it is described herein that by introducing random or semi-random mutations in the loops connecting beta strands A-B, C'-D and E-F of a human or humanized variable TCR-domain, mutated domains can be selected that bind specifically to either human serum albumin or to Fc-receptors, which are not normally recognized and bound by human TCR domains. The mutations introduced include mutations in which selected amino acid residues in the wild-type sequence were replaced by randomly chosen residues, and they also include insertions of extra amino acid residues in the loops mentioned above.

By analogy the domains from any class of T-cell receptors and from T-cell receptors from any species are amenable to this type of engineering. Furthermore not only the specific loops targeted in the examples of the present invention can be manipulated, but any structural loop connecting beta strands in T-cell receptor domains can be manipulated in the same way.

Engineered T-cell receptor domains from any organism and from any class can be used either as such (as single domains), or as part of a larger molecule. For example, they can be part of an intact T-cell receptor, which accordingly would have its "normal" antigen binding region formed by the 6 CDRs and the new, engineered antigen binding region. Like this, a multi-specific, e.g. bispecific, T-cell receptor could be generated. The engineered T-cell receptor domains can also be part of any fusion protein. The use of these engineered T-cell receptor domains is in the general field of the use of T-cell receptors and immunoglobulins.

The following examples shall explain the present invention in more detail without, however, restricting it.

### Examples:

### Example 1

A number of different libraries are constructed based on a soluble version of the 1G4 TCR, which is specific for the NY-ESO epitope (WO02005113595).

The library genes are assembled from specific synthetic oligonucleotides and cloned as full length TCR alpha and beta chains displayed on filamentous phage. The alpha chain is expressed in soluble format, and the beta chain is expressed as in-frame fusion to the geneIII coat protein of M13 bacteriophage. The alpha chain has a non-native Cystein residue(encoded by the mutation Thr84Cys (IMGT numbering)) and the beta chain has a non-native Cystein residue (encoded by the mutation Ser79Cys(IMGT numbering)) to allow heterodimer formation. Cloning, selection and characterization can be done as described in Li et al. (2005) Nat Biotechnol. 23:349-354. The following 1G4 TCR gene and library gene pairs are cloned into the three-cistron phage display vector, pEX746 essentially as described in Li et al. (2005) Nat Biotechnol. 23:349-354.
a) 1G4 alpha-chain wild type gene in combination with V-beta 1G4-1 library gene
b) 1G4 alpha-chain wild type gene in combination with V-beta 1G4-2 library gene
c) 1G4 alpha-chain wild type gene in combination with C-beta 1G4-1 library gene
d) 1G4 alpha-chain wild type gene in combination with C-beta 1G4-2 library gene
e) 1G4 beta-chain wild type gene in combination with V-alpha 1G4-1 library gene
f) 1G4 beta-chain wild type gene in combination with V-alpha 1G4-2 library gene
g) 1G4 beta-chain wild type gene in combination with C-alpha 1G4-1 library gene
h) 1G4 beta-chain wild type gene in combination with C-alpha 1G4-2 library gene

Below are the sequences for 1G4 wild type chains and for libraries in both alpha and beta chain, with variable and constant domains to be randomized for each chain separately. For each, Valpha, Calpha, Vbeta and Cbeta, two libraries are given: one with just replacements, one with additional insertions. That means that a total of 8 libraries are described below.

Gene of 1G4 alpha-chain wild type, Genbank accession no. CS230225 (including start codon and stop codon):

1 ATGCAGGAGG TGACACAGAT TCCTGCAGCT CTGAGTGTCC CAGAAGGAGA
AAACTTGGTT TCAACTGCA GTTTCACTGA TAGCGCTATT TACAACCTCC
101 AGTGGTTTAG GCAGGACCCT GGGAAAGGTC TCACATCTCT GTTGCTTATT
CAGTCAAGTC AGAGAGAGCA AACAAGTGGA AGACTTAATG CCTCGCTGGA
201 TAAATCATCA GGACGTAGTA CTTTATACAT TGCAGCTTCT CAGCCTGGTG
ACTCAGCCAC CTACCTCTGT GCTGTGAGGC CCACATCAGG AGGAAGCTAC
301 ATACCTACAT TTGGAAGAGG AACCAGCCTT ATTGTTCATC CGTATATCCA
GAACCCTGAC CCTGCCGTGT ACCAGCTGAG AGACTCTAAA TCCAGTGACA
401 AGTCTGTCTG CCTATTCACC GATTTTGATT CTCAAACAAA TGTGTCACAA
AGTAAGGATT CTGATGTGTA TATCACAGAC AAATGTGTGC TAGACATGAG
501 GTCTATGGAC TTCAAGAGCA ACAGTGCTGT GGCCTGGAGC AACAAATCTG
ACTTTGCATG TGCAAACGCC TTCAACAACA GCATTATTCC AGAAGACACC
601 TTCTTCCCCA GCCCAGAAAG TTCCTAA

Protein of 1G4 alpha-chain wild type, Genbank accession no. CS230225 (including start codon and stop codon):

MQEVTQIPAALSVPEGENLVLNCSFTDSAIYNLQWFRQDPGKGLTSLLLIQSSQREQTSGRLNA
SLDKSSGRSTLYIAASQPGDSATYLCAVRPTSGGSYIPTFGRGTSLIVHPYIQNPDPAVYQLRD
SKSSDKSVCLFTDFDSQTNVSQSKDSDVYITDKCVLDMRSMDFKSNSAVAWSNKSDFACANAFN
NSIIPEDTFFPSPESS#

Gene of V-alpha-library 1G4-1 (including start codon and stop codon):

1 ATGCAGGAGG TGACACAGAT TCCTGCAGCT CTGAGTGTCC CANNSNNSNN
SNNSTTGGTT CTCAACTGCA GTTTCACTGA TAGCGCTATT TACAACCTCC
101 AGTGGTTTAG GCAGGACCCT GGGAAAGGTC TCACATCTCT GTTGCTTATT
CAGTCAAGTC AGAGAGAGCA AACAAGTGGA AGACTTAATG CCTCGCTGGA

201 TAAATCATCA GGACGTAGTA CTTTATACAT TNNSNNSNNS NNSCCTGGTG
ACTCAGCCAC CTACCTCTGT GCTGTGAGGC CCACATCAGG AGGAAGCTAC
301 ATACCTACAT TTGGAAGAGG AACCAGCCTT ATTGTTCATC CGTATATCCA
GAACCCTGAC CCTGCCGTGT ACCAGCTGAG AGACTCTAAA TCCAGTGACA
401 AGTCTGTCTG CCTATTCACC GATTTTGATT CTCAAACAAA TGTGTCACAA
AGTAAGGATT CTGATGTGTA TATCACAGAC AAATGTGTGC TAGACATGAG
501 GTCTATGGAC TTCAAGAGCA ACAGTGCTGT GGCCTGGAGC AACAAATCTG
ACTTTGCATG TGCAAACGCC TTCAACAACA GCATTATTCC AGAAGACACC
601 TTCTTCCCCA GCCCAGAAAG TTCCTAA

Protein of V-alpha-library 1G4-1 (including start codon and stop codon):

MQEVTQIPAALSVPXXXXLVLNCSFTDSAIYNLQWFRQDPGKGLTSLLLIQSSQREQTSGRLNA
SLDKSSGRSTLYIXXXXPGDSATYLCAVRPTSGGSYIPTFGRGTSLIVHPYIQNPDPAVYQLRD
SKSSDKSVCLFTDFDSQTNVSQSKDSDVYITDKCVLDMRSMDFKSNSAVAWSNKSDFACANAFN
NSIIPEDTFFPSPESS#

### Mutated residues:

EGEN 15-18
AASQ 92-95

Gene of V-alpha-library 1G4-2 (including start codon and stop codon):

1 ATGCAGGAGG TGACACAGAT TCCTGCAGCT CTGAGTGTCC CANNSNNSNN
SNNSTTGGTT CTCAACTGCA GTTTCACTGA TAGCGCTATT TACAACCTCC
101 AGTGGTTTAG GCAGGACCCT GGGAAAGGTC TCACATCTCT GTTGCTTATT
CAGTCAAGTC AGAGAGAGCA AACAAGTNNS NNSNNSAATG CCTCGCTGGA
201 TAAATCATCA GGACGTAGTA CTTTATACAT TNNSNNSNNS NNSCCTGGTG
ACTCAGCCAC CTACCTCTGT GCTGTGAGGC CCACATCAGG AGGAAGCTAC
301 ATACCTACAT TTGGAAGAGG AACCAGCCTT ATTGTTCATC CGTATATCCA
GAACCCTGAC CCTGCCGTGT ACCAGCTGAG AGACTCTAAA TCCAGTGACA
401 AGTCTGTCTG CCTATTCACC GATTTTGATT CTCAAACAAA TGTGTCACAA
AGTAAGGATT CTGATGTGTA TATCACAGAC AAATGTGTGC TAGACATGAG

501 GTCTATGGAC TTCAAGAGCA ACAGTGCTGT GGCCTGGAGC AACAAATCTG
ACTTTGCATG TGCAAACGCC TTCAACAACA GCATTATTCC AGAAGACACC
601 TTCTTCCCCA GCCCAGAAAG TTCCTAA

Protein of V-alpha-library 1G4-2 (including start codon and stop codon):

MQEVTQIPAALSVPXXXXLVLNCSFTDSAIYNLQWFRQDPGKGLTSLLLIQSSQREQTSXXXNA
SLDKSSGRSTLYIXXXXPGDSATYLCAVRPTSGGSYIPTFGRGTSLIVHPYIQNPDPAVYQLRD
SKSSDKSVCLFTDFDSQTNVSQSKDSDVYITDKCVLDMRSMDFKSNSAVAWSNKSDFACANAFN
NSIIPEDTFFPSPESS#

### Mutated residues:

EGEN 15-18
GRL 70, 78,79
AASQ 92-95
Gene of C-alpha-library 1G4-1 (including start codon and stop codon):

DNA sequence with translation:

1 ATGCAGGAGG TGACACAGAT TCCTGCAGCT CTGAGTGTCC CAGAAGGAGA
AAACTTGGTT CTCAACTGCA GTTTCACTGA TAGCGCTATT TACAACCTCC
101 AGTGGTTTAG GCAGGACCCT GGGAAAGGTC TCACATCTCT GTTGCTTATT
CAGTCAAGTC AGAGAGAGCA AACAAGTGGA AGACTTAATG CCTCGCTGGA
201 TAAATCATCA GGACGTAGTA CTTTATACAT TGCAGCTTCT CAGCCTGGTG
ACTCAGCCAC CTACCTCTGT GCTGTGAGGC CCACATCAGG AGGAAGCTAC
301 ATACCTACAT TTGGAAGAGG AACCAGCCTT ATTGTTCATC CGTATATCCA
GAACCCTGAC CCTGCCGTGT ACCAGCTGAG AGACTCTAAA TCCAGTGACA
401 AGTCTGTCTG CCTATTCACC GATTTTGATT CTCAAACAAA TGTGTCACAA
AGTNNSNNSN NSNNSGTGTA TATCACAGAC AAATGTGTGC TAGACATGAG
501 GTCTATGGAC TTCAAGAGCA ACAGTGCTGT GGCCTGGAGC NNSNNSNNSN
NSTTTGCATG TGCAAACGCC TTCAACAACA GCATTATTCC AGAAGACACC
601 TTCTTCCCCA GCCCAGAAAG TTCCTAA

Protein of C-alpha-library 1G4-1 (including start codon and stop codon

MQEVTQIPAALSVPEGENLVLNCSFTDSAIYNLQWFRQDPGKGLTSLLLIQSSQREQTSGRLNA
SLDKSSGRSTLYIAASQPGDSATYLCAVRPTSGGSYIPTFGRGTSLIVHPYIQNPDPAVYQLRD
SKSSDKSVCLFTDFDSQTNVSQSXXXXVYITDKCVLDMRSMDFKSNSAVAWSXXXXFACANAFN
NSIIPEDTFFPSPESS#

### Mutated residues:

KDSD 43-77
NKSD 91-101
Gene of C-alpha-library 1G4-2 (including start codon and stop codon): 
1 ATGCAGGAGG TGACACAGAT TCCTGCAGCT CTGAGTGTCC CAGAAGGAGA
AAACTTGGTT CTCAACTGCA GTTTCACTGA TAGCGCTATT TACAACCTCC
101 AGTGGTTTAG GCAGGACCCT GGGAAAGGTC TCACATCTCT GTTGCTTATT
CAGTCAAGTC AGAGAGAGCA AACAAGTGGA AGACTTAATG CCTCGCTGGA
201 TAAATCATCA GGACGTAGTA CTTTATACAT TGCAGCTTCT CAGCCTGGTG
ACTCAGCCAC CTACCTCTGT GCTGTGAGGC CCACATCAGG AGGAAGCTAC
301 ATACCTACAT TTGGAAGAGG AACCAGCCTT ATTGTTCATC CGTATATCCA
GAACCCTGAC CCTGCCGTGT ACCAGCTGAG AGACTCTAAA TCCAGTGACA
401 AGTCTGTCTG CCTATTCACC GATTTTGATT CTCAAACAAA TGTGTCACAA
AGTNNSNNSN NSNNSNNSGT GTATATCACA GACAAATGTG TGCTAGACAT
501 GAGGTCTATG GACTTCAAGA GCAACAGTGC TGTGGCCTGG AGCNNSNNSN
NSNNSNNSTT TGCATGTGCA AACGCCTTCA ACAACAGCAT TATTCCAGAA
601 GACACCTTCT TCCCCAGCCC AGAAAGTTCC TAA
Protein of C-alpha-library 1G4-2 (including start codon and stop codon):

MQEVTQIPAALSVPEGENLVLNCSFTDSAIYNLQWFRQDPGKGLTSLLLIQSSQREQTSGRLNA
SLDKSSGRSTLYIAASQPGDSATYLCAVRPTSGGSYIPTFGRGTSLIVHPYIQNPDPAVYQLRD
SKSSDKSVCLFTDFDSQTNVSQSXXXXXVYITDKCVLDMRSMDFKSNSAVAWSXXXXXFACANA
FNNSIIPEDTFFPSPESS#

### Mutated residues:

Inserted residues "X"
KDSXD
NKSXD
Gene of V-beta 1G4 wild type, GenBank Accession no. CS230226 (including start codon and stop codon): 
1     atgggtgtca ctcagacccc aaaattccag gtcctgaaga caggacagag
      catgacactg
61    cagtgtgccc aggatatgaa ccatgaatac atgtcctggt atcgacaaga
      cccaggcatg
121   gggctgaggc tgattcatta ctcagttggt gctggtatca ctgaccaagg
      agaagtcccc
181   aatggctaca atgtctccag atcaaccaca gaggatttcc cgctcaggct
      gctgtcggct
241   gctccctccc agacatctgt gtacttctgt gccagcagtt acgtcgggaa
      caccggggag
301   ctgtttttg gagaaggctc taggctgacc gtactggagg acctgaaaaa
      cgtgttccca
361   cccgaggtcg ctgtgtttga gccatcagaa gcagagatct cccacaccca
      aaaggccaca
421   ctggtgtgcc tggccacagg cttctacccc gaccacgtgg agctgagctg
      gtgggtgaat
481   gggaaggagg tgcacagtgg ggtctgcaca gacccgcagc ccctcaagga
      gcagcccgcc
541   ctcaatgact ccagatacgc tctgagcagc cgcctgaggg tctcggccac
      cttctggcag
601   gacccccgca accacttccg ctgtcaagtc cagttctacg ggctctcgga
      gaatgacgag
661   tggacccagg atagggccaa acccgtcacc cagatcgtca gcgccgaggc
      ctggggtaga
721   gcagactaa
Protein of V-beta 1G4 wild type, GenBank Accession no. CS230226 (including start codon and stop codon): 
MGVTQTPKFQVLKTGQSMTLQCAQDMNHEYMSWYRQDPGMGLRLIHYSVGAGITDQGEVPNGYN
VSRSTTEDFPLRLLSAAPSQTSVYFCASSYVGNTGELFFGEGSRLTVLEDLKNVFPPEVAVFEP
 
SEAEISHTQKATLVCLATGFYPDHVELSWWVNGKEVHSGVCTDPQPLKEQPALNDSRYALSSRL
RVSATFWQDPRNHFRCQVQFYGLSENDEWTQDRAKPVTQIVSAEAWGRAD#
Gene of V-beta-library 1G4-1 (including start codon and stop codon): 
1 ATGGGTGTCA CTCAGACCCC AAAATTCCAG GTCCTGNNSN NSNNSNNSNN
SATGACACTG CAGTGTGCCC AGGATATGAA CCATGAATAC ATGTCCTGGT
101 ATCGACAAGA CCCAGGCATG GGGCTGAGGC TGATTCATTA CTCAGTTGGT
GCTGGTATCA CTGACCAAGG AGAAGTCCCC AATGGCTACA ATGTCTCCAG
201 ATCAACCACA GAGGATTTCC CGCTCAGGCT GNNSNNSNNS NNSCCCTCCC
AGACATCTGT GTACTTCTGT GCCAGCAGTT ACGTCGGGAA CACCGGGGAG
301 CTGTTTTTTG GAGAAGGCTC TAGGCTGACC GTACTGGAGG ACCTGAAAAA
CGTGTTCCCA CCCGAGGTCG CTGTGTTTGA GCCATCAGAA GCAGAGATCT
401 CCCACACCCA AAAGGCCACA CTGGTGTGCC TGGCCACAGG CTTCTACCCC
GACCACGTGG AGCTGAGCTG GTGGGTGAAT GGGAAGGAGG TGCACAGTGG
501 GGTCTGCACA GACCCGCAGC CCCTCAAGGA GCAGCCCGCC CTCAATGACT
CCAGATACGC TCTGAGCAGC CGCCTGAGGG TCTCGGCCAC CTTCTGGCAG
601 GACCCCCGCA ACCACTTCCG CTGTCAAGTC CAGTTCTACG GGCTCTCGGA
GAATGACGAG TGGACCCAGG ATAGGGCCAA ACCCGTCACC CAGATCGTCA
701 GCGCCGAGGC CTGGGGTAGA GCAGACTAA
Protein of V-beta-library 1G4-1 (including start codon and stop codon):

MGVTQTPKFQVLXXXXMTLQCAQDMNHEYMSWYRQDPGMGLRLIHYSVGAGITDQGEVPNGYN
VSRSTTEDFPLRLXXXXPSQTSVYFCASSYVGNTGELFFGEGSRLTVLEDLKNVFPPEVAVFEP
SEAEISHTQKATLVCLATGFYPDHVELSWWVNGKEVHSGVCTDPQPLKEQPALNDSRYALSSRL
RVSATFWQDPRNHFRCQVQFYGLSENDEWTQDRAKPVTQIVSAEAWGRAD

### Mutated residues:

KTGQS 15-18.
LSAA 92-95
Gene of V-beta-library 1G4-2 (including start codon and stop codon): 
1 ATGGGTGTCA CTCAGACCCC AAAATTCCAG GTCCTGNNSN NSNNSNNSNN
SATGACACTG CAGTGTGCCC AGGATATGAA CCATGAATAC ATGTCCTGGT
101 ATCGACAAGA CCCAGGCATG GGGCTGAGGC TGATTCATTA CTCAGTTGGT
GCTGGTATCA CTGACCAAGG AGAAGTCCCC AATNNSTACN NSNNSTCCAG
201 ATCAACCACA GAGGATTTCC CGCTCAGGCT GNNSNNSNNS NNSCCTCCC
AGACATCTGT GTACTTCTGT GCCAGCAGTT ACGTCGGGAA CACCGGGGAG
301 CTGTTTTTTG GAGAAGGCTC TAGGCTGACC GTACTGGAGG ACCTGAAAAA
CGTGTTCCCA CCCGAGGTCG CTGTGTTTGA GCCATCAGAA GCAGAGATCT
401 CCCACACCCA AAAGGCCACA CTGGTGTGCC TGGCCACAGG CTTCTACCCC
GACCACGTGG AGCTGAGCTG GTGGGTGAAT GGGAAGGAGG TGCACAGTGG
501 GGTCTGCACA GACCCGCAGC CCCTCAAGGA GCAGCCCGCC CTCAATGACT
CCAGATACGC TCTGAGCAGC CGCCTGAGGG TCTCGGCCAC CTTCTGGCAG
601 GACCCCCGCA ACCACTTCCG CTGTCAAGTC CAGTTCTACG GGCTCTCGGA
GAATGACGAG TGGACCCAGG ATAGGGCCAA ACCCGTCACC CAGATCGTCA
701 GCGCCGAGGC CTGGGGTAGA GCAGACTAA
Protein of V-beta-library 1G4-2 (including start codon and stop codon):

MGVTQTPKFQVLXXXXMTLQCAQDMNHEYMSWYRQDPGMGLRLIHYSVGAGITDQGEVPNXYX
XSRSTTEDFPLRLXXXXPSQTSVYFCASSYVGNTGELFFGEGSRLTVLEDLKNVFPPEVAVFEP
SEAEISHTQKATLVCLATGFYPDHVELSWWVNGKEVHSGVCTDPQPLKEQPALNDSRYALSSRL
RVSATFWQDPRNHFRCQVQFYGLSENDEWTQDRAKPVTQIVSAEAWGRAD

### Mutated residues:

KTGQS 15-18
G, N, V 75, 77, 78
LSAA 92-95
Gene of C-beta-library 1G4-1 (including start codon and stop codon): 
1 ATGGGTGTCA CTCAGACCCC AAAATTCCAG GTCCTGAAGA CAGGACAGAG
CATGACACTG CAGTGTGCCC AGGATATGAA CCATGAATAC ATGTCCTGGT
101 ATCGACAAGA CCCAGGCATG GGGCTGAGGC TGATTCATTA CTCAGTTGGT
GCTGGTATCA CTGACCAAGG AGAAGTCCCC AATGGCTACA ATGTCTCCAG
 
201 ATCAACCACA GAGGATTTCC CGCTCAGGCT GCTGTCGGCT GCTCCCTCCC
AGACATCTGT GTACTTCTGT GCCAGCAGTT ACGTCGGGAA CACCGGGGAG
301 CTGTTTTTTG GAGAAGGCTC TAGGCTGACC GTACTGGAGG ACCTGAAAAA
CGTGTTCCCA CCCGAGGTCG CTGTGTTTGA GCCATCAGAA GCAGAGNNSN
401 NSNNSNNSNN SAAGGCCACA CTGGTGTGCC TGGCCACAGG CTTCTACCCC
GACCACGTGG AGCTGAGCTG GTGGGTGAAT GGGAAGGAGG TGCACAGTGG
501 GGTCTGCACA GACCCGCAGC CCCTCAAGGA GCAGCCCGCC CTCAATGACT
CCAGATACGC TCTGAGCAGC CGCCTGAGGG TCNNSNNSNN SNNSTGGNNS
601 GACCCCCGCA ACCACTTCCG CTGTCAAGTC CAGTTCTACG GGCTCTCGGA
GAATGACGAG TGGACCCAGG ATAGGGCCAA ACCCGTCACC CAGATCGTCA
701 GCGCCGAGGC CTGGGGTAGA GCAGACTAA
Gene of C-beta-library 1G4-1 (including start codon and stop codon):

MGVTQTPKFQVLKTGQSMTLQCAQDMNHEYMSWYRQDPGMGLRLIHYSVGAGITDQGEVPNGYN
VSRSTTEDFPLRLLSAAPSQTSVYFCASSYVGNTGELFFGEGSRLTVLEDLKNVFPPEVAVFEP
SEAEXXXXXKATLVCLATGFYPDHVELSWWVNGKEVHSGVCTDPQPLKEQPALNDSRYALSSRL
RVXXXXWXDPRNHFRCQVQFYGLSENDEWTQDRAKPVTQIVSAEAWGRAD

### Mutated residues:

ISHTQ 14, 15, 15.1, 16, 17
SATFQ 92-95, 96.1
Gene of C-beta-library 1G4-2 (including start codon and stop codon): 
1     ATGGGTGTCA CTCAGACCCC AAAATTCCAG GTCCTGAAGA CAGGACAGAG
      CATGACACTG CAGTGTGCCC AGGATATGAA CCATGAATAC ATGTCCTGGT
101   ATCGACAAGA CCCAGGCATG GGGCTGAGGC TGATTCATTA CTCAGTTGGT
      GCTGGTATCA CTGACCAAGG AGAAGTCCCC AATGGCTACA ATGTCTCCAG
201   ATCAACCACA GAGGATTTCC CGCTCAGGCT GCTGTCGGCT GCTCCCTCCC
      AGACATCTGT GTACTTCTGT GCCAGCAGTT ACGTCGGGAA CACCGGGGAG
301   CTGTTTTTTG GAGAAGGCTC TAGGCTGACC GTACTGGAGG ACCTGAAAAA
      CGTGTTCCCA CCCGAGGTCG CTGTGTTTGA GCCATCAGAA GCAGAGNNSN
 
401 NSNNSNNSNN SNNSAAGGCC ACACTGGTGT GCCTGGCCAC AGGCTTCTAC
CCCGACCACG TGGAGCTGAG CTGGTGGGTG AATGGGAAGG AGGTGCACAG
501 TGGGGTCTGC ACAGACCCGC AGCCCCTCAA GGAGCAGCCC GCCCTCAATG
ACTCCAGATA CGCTCTGAGC AGCCGCCTGA GGGTCNNSNN SNNSNNSNNS
601 TGGNNSGACC CCCGCAACCA CTTCCGCTGT CAAGTCCAGT TCTACGGGCT
CTCGGAGAAT GACGAGTGGA CCCAGGATAG GGCCAAACCC GTCACCCAGA
701 TCGTCAGCGC CGAGGCCTGG GGTAGAGCAG ACTAA
Protein of C-beta-library 1G4-2 (including start codon and stop codon):

MGVTQTPKFQVLKTGQSMTLQCAQDMNHEYMSWYRQDPGMGLRLIHYSVGAGITDQGEVPNGYN
VSRSTTEDFPLRLLSAAPSQTSVYFCASSYVGNTGELFFGEGSRLTVLEDLKNVFPPEVAVFEP
SEAEXXXXXXKATLVCLATGFYPDHVELSWWVNGKEVHSGVCTDPQPLKEQPALNDSRYALSSR
LRVXXXXXWXDPRNHFRCQVQFYGLSENDEWTQDRAKPVTQIVSAEAWGRAD

### Mutated residues:

Inserted residues "X"
ISHXTQ 14, 15, 15.1, 16, 17
SATXFQ 92-95, 96.1

### Example 2:

After ligation of the library inserts into the vector, the steps of phage preparation are performed following standard protocols. Briefly, the ligation mixtures are transformed into *E. coli* TGJ cells by electroporation. Subsequently, phage particles are rescued from *E. coli* TG1 cells with helper phage M13-KO7. Phage particles are then precipitated from culture supernatant with PEG/NaCl in 2 steps, dissolved in water and used for selectior by panning or, alternatively, they were stored at minus 80 °C.

### Selection of clones binding specifically to Human serum albumin:

The libraries as described in example 1 are used in panning rounds for the isolation of specifically binding clones following standard protocols. Briefly, the phage libraries are suspended in binding buffer (PBS, 1% ovalbumin, 0.005% Tween 20) and panned against human serum albumin immobilized directly on maxisorp plates ( 10 micrograms/ml in PBS, overnight at 4°C; plates are blocked with Blocker Casein (Pierce). After 2 hours, unbound phage are removed by repetitive washing (PBS, 0.05% Tween 20) and bound phage are eluted with 500 mM KC1, 10mM HCl, pH2. 2, 3, 4 or 5 such panning rounds are performed. After each panning round on human serum albumin, the resulting clones are selected or tested for binding to human serum albumin.

### Selection of clones binding specifically to FcRn:

The panning is performed as described in WO02060919, Example 6.2. In short, phage libraries are resuspended in 5 ml 20 mM MES, pH 6.0/5% skimmed milk/0.05% Tween 20 and added (100 microlitre of 5 x 10¹² PFU/ml/well) to 20 wells of a Maxisorp immunoplate (Nunc) previously coated with 1 microgram of murine FcRn and blocked with 5% skimmed milk. After incubation for 2 h at 37°C, wells are washed 10-30 times with 20 mM MES, pH 6.0/0.2% Tween 20/0.3 M NaCl and phage eluted by incubation in 100 microlitre PBS, pH 7.4/well for 30 min at 37°C. Phage are used to reinfect exponentially growing E. coli TG1. 2, 3, 4 or 5 such panning rounds are performed.
After each panning round on FcRn the resulting clones are selected or tested for binding to FcRn.

### Selection of clones binding specifically to Fc-gamma receptors:

Panning against Fc-gammaRI, Fc-gammaRIIA, Fc-gammaRIIB and Fc-gammaRIIIB are performed as described in Berntzen et al (2006) Protein Eng Des Sel 19:121-128. Briefly, cultured cells which are transfected with the genes coding for Fc-gammaRI, Fc-gammaRIIA, Fc-gammaRIIB or Fc-gammaRIIIB are used as targets for the selection of phage libraries. Cells which naturally express Fc-gammaRI, Fc-gammaRIIA, Fc-gammaRIIB or Fc-gammaRIIIB can also be used for this purpose. E.g. the cell line U937 (American Type Culture Collection CRL-1503) which constitutively expresses FcgRI, FcgRIIA and FcgRIIB can be used as a target. The level of FcgRI can be upregulated in this cell line by IFN-g stimulation, making this receptor the most abundant of the FcgRs. Soluble versions of the receptors, which can be produced recombinantly in bacteria, yeast or animal cells can also be used for this purpose.

After each panning round on Fc-gammaRI, Fc-gammaRIIA, Fc-gammaRIIB or Fc-gammaRIIIB the resulting clones are selected or tested for binding to the respective receptor e.g. by ELISA or flow cytometry.

## Claims

1. Method for engineering a T-cell receptor domain polypeptide comprising a structural loop region to introduce a new antigen binding site into said structural loop region, wherein the structural loop is a non-CDR loop, and determining the binding to an epitope of said antigen, wherein the unmodified T-cell receptor domain polypeptide does not significantly bind to said epitope of said antigen comprising the steps of:
- providing a nucleic acid encoding a T-cell receptor domain polypeptide comprising at least one structural loop region,
- modifying at least one nucleotide residue of at least one of said structural loop regions by site-directed random mutation, wherein two or more specific amino acid residues of the loops are exchanged or introduced using randomly generated inserts into such structural loops,
- transferring said modified nucleic acid in an expression system,
- expressing said modified T-cell receptor domain polypeptide,
- contacting the expressed modified T-cell receptor domain polypeptide with said epitope, and
- determining whether said modified T-cell receptor domain polypeptide binds to said epitope.

2. Method according to claim 1, wherein said T-cell receptor domain polypeptide binds specifically to at least two epitopes.

3. Method according to any one of claims 1 and 2, **characterized in that** the T- cell receptor domain polypeptide is of human or murine origin.

4. Method according to any one of claims 1 to 3, **characterized in that** the T-cell receptor domain polypeptide is derived from a T-cell receptor domain selected from the variable or constant domain, preferably from the group consisting of V-alpha, V-beta, V-gamma, V-delta, C-alpha, C-beta, C-gamma, C-delta.

5. Method according to any one of claims 1 to 4, **characterized in that** the modified loop regions of the variable domains comprise at least one modification within amino acids 11 to 19, amino acids 43 to 51, amino acids 67 to 80 or amino acids 90 to 99, where the numbering of the amino acid positions of the domains is that of the ImMunoGeneTics (IMGT) numbering scheme.

6. Method according to any one of claims 1 to 4, **characterized in that** the modified loop regions of the constant domains comprise at least one modification within amino acids 9 to 20, amino acids 27 to 36, amino acids 41 to 78, amino acids 82 to 85, amino acids 90 to 102 or amino acids 107 to 116, where the numbering of the amino acid position of the domains is that of the IMGT numbering scheme.

7. Method according to any one of the claims 1 to 6, **characterized in that** the modification of at least one nucleotide of a nucleic acid results in a substitution, deletion and/or insertion of one or more amino acids of the T-cell receptor domain polypeptide encoded by said nucleic acid.

8. Method according to any one of claims 1 to 7, **characterized in that** at least one amino acid of at least one structural loop region is modified by site-directed random mutation.

9. Method according to claim 8, **characterized in that** the randomly modified nucleic acid molecule comprises at least one nucleotide repeating unit having the coding sequence NNS, NNN, NNK, TMT, WMT, RMC, RMG, MRT, SRC, KMT, RST, YMT, MKC, RSA, RRC, where the coding is according to IUPAC.

## Patentansprüche

1. Verfahren zum Bearbeiten eines T-Zellen-Rezeptordomänenpolypeptids, das eine strukturelle Schleifenregion umfasst, um eine neue Antigenbindungsstelle in die strukturelle Schleifenregion einzuführen, worin die strukturelle Schleife keine CDR-Schleife ist, und zum Bestimmen der Bindung an ein Epitop des Antigens, worin das unmodifizierte T-Zellen-Rezeptordomänenpolypeptid nicht signifikant an das Epitop des Antigens bindet, das die folgenden Schritte umfasst:
- Bereitstellen einer Nucleinsäure, die für ein T-Zellen-Rezeptordomänenpolypeptid mit zumindest einer strukturellen Schleifenregion kodiert,
- Modifizieren zumindest eines Nucleotidrests von zumindest einer der strukturellen Schleifenregionen durch stellengerichtete Zufallsmutagenese, worin zwei oder mehrere spezifische Aminosäurereste der Schleifen unter Verwendung von zufällig erstellten Inserts in solche strukturellen Schleifen ausgetauscht oder eingeführt werden,
- Transferieren der modifizierten Nucleinsäure in ein Expressionssystem,
- Exprimieren des modifizierten T-Zellen-Rezeptordomänenpolypeptids,
- Kontaktieren des exprimierten modifizierten T-Zellen-Rezeptordomänenpolypeptids mit dem Epitop und
- Bestimmen, ob das modifizierte T-Zellen-Rezeptordomänenpolypeptid an das Epitop bindet.

2. Verfahren nach Anspruch 1, worin das T-Zellen-Rezeptordomänenpolypeptid spezifisch an zumindest zwei Epitope bindet.

3. Verfahren nach einem der Ansprüche 1 und 2, **dadurch gekennzeichnet, dass** das T-Zellen-Rezeptordomänenpolypeptid menschlichen oder murinen Ursprungs ist.

4. Verfahren nach einem der Ansprüche 1 bis 3, **dadurch gekennzeichnet, dass** das T-Zellen-Rezeptordomänenpolypeptid von einer T-Zellen-Rezeptordomäne abgeleitet ist, die aus der variablen oder konstanten Domäne, vorzugsweise aus der Gruppe bestehend aus V-alpha, V-beta, V-gamma, V-delta, C-alpha, C-beta, C-gamma, C-delta, ausgewählt ist.

5. Verfahren nach einem der Ansprüche 1 bis 4, **dadurch gekennzeichnet, dass** die modifizierten Schleifenregionen der variablen Domänen zumindest eine Modifikation innerhalb Aminosäuren 11 bis 19, Aminosäuren 43 bis 51, Aminosäuren 67 bis 80 oder Aminosäuren 90 bis 99 umfassen, wobei die Nummerierung der Aminosäurepositionen der Domänen nach dem ImMunoGeneTics- (IMGT-) Nummerierungssystem vorgenommen wurde.

6. Verfahren nach einem der Ansprüche 1 bis 4, **dadurch gekennzeichnet, dass** die modifizierten Schleifenregionen der konstanten Domänen zumindest eine Modifikation innerhalb der Aminosäuren 9 bis 20, Aminosäuren 27 bis 36, Aminosäuren 41 bis 78, Aminosäuren 82 bis 85, Aminosäuren 90 bis 102 oder Aminosäuren 107 bis 116 umfassen, wobei die Nummerierung der Aminosäureposition der Domänen nach dem IMGT-Nummerierungssystem vorgenommen wurde.

7. Verfahren nach einem der Ansprüche 1 bis 6, **dadurch gekennzeichnet, dass** die Modifikation von zumindest einem Nucleotid einer Nucleinsäure zu einer Substitution, Deletion und/oder Insertion einer oder mehrerer Aminosäuren des von der Nucleinsäure kodierten T-Zellen-Rezeptordomänenpolypeptids führt.

8. Verfahren nach einem der Ansprüche 1 bis 7, **dadurch gekennzeichnet, dass** zumindest eine Aminosäure von zumindest einer strukturellen Schleifenregion durch stellengerichtete Zufallsmutation modifiziert ist.

9. Verfahren nach Anspruch 8, **dadurch gekennzeichnet, dass** das zufällig modifizierte Nucleinsäuremolekül zumindest eine Nucleotid-Wiederholungseinheit mit der Kodiersequenz NNS, NNN, NNK, TMT, WMT, RMC, RMG, MRT, SRC, KMT, RST, YMT, MKC, RSA, RRC umfasst, wobei die Kodierung nach IUPAC erfolgt.

## Revendications

1. Procédé pour synthétiser un polypeptide de domaine de récepteur de cellules T comprend une région de boucle structurelle pour introduire un nouveau site de liaison d'antigène dans ladite région de boucle structurelle, où la boucle structurelle est une boucle non-CDR, et déterminer la liaison à un épitope dudit antigène, où le polypeptide de domaine de récepteur de cellules T non modifié ne se lie pas d'une manière significative audit épitope dudit antigène, comprenant les étapes de :
- réaliser un acide nucléique codant pour un polypeptide de domaine de récepteur de cellules T comprenant au moins une région de boucle structurelle,
- modifier au moins un résidu de nucléotide d'au moins une desdites régions de boucle structurelle par une mutation aléatoire dirigée au site, où deux ou plusieurs résidus d'acide aminé spécifiques des boucles sont échangés ou introduits en utilisant des inserts produits d'une manière aléatoire dans de telles boucles structurelles,
- transférer ledit acide nucléique modifié dans un système d'expression,
- exprimer ledit polypeptide de domaine de récepteur de cellules T modifié,
- mettre en contact le polypeptide de domaine de récepteur de cellules T modifié avec ledit épitope, et
- déterminer si ledit polypeptide de domaine de récepteur de cellules T modifié se lie audit épitope.

2. Procédé selon la revendication 1, dans lequel ledit polypeptide de domaine de récepteur de cellules T se lie spécifiquement à au moins deux épitopes.

3. Procédé selon l'une quelconque des revendications 1 et 2, **caractérisé en ce que** le polypeptide de domaine de récepteur de cellules T est d'origine humaine ou murine.

4. Procédé selon l'une quelconque des revendications 1 à 3, **caractérisé en ce que** le polypeptide de domaine de récepteur de cellules T est dérivé d'un domaine de récepteur de cellules T sélectionné dans le domaine variable ou constant, de préférence du groupe consistant en V-alpha, V-béta, V-gamma, V-delta, C-alpha, C-béta, C-gamma, C-delta.

5. Procédé selon l'une quelconque des revendications 1 à 4, **caractérisé en ce que** les régions de boucle modifiées des domaines variables comprennent au moins une modification dans les acides aminés 11 à 19, acides aminés 43 à 51, acides aminés 67 à 80 ou acides aminés 90 à 99, où la numérotation des positions d'acide aminé des domaines est celle du schéma de numérotation ImMunoGeneTics (IMGT).

6. Procédé selon l'une quelconque des revendications 1 à 4, **caractérisé en ce que** les régions de boucle modifiées des domaines constants comprennent au moins une modification dans les acides aminés 9 à 20, acides aminés 27 à 36, acides aminés 41 à 78, acides aminés 82 à 85, acides aminés 90 à 102 ou acides aminés 107 à 116, où la numérotation de la position d'acide aminé des domaines est celle du schéma de numérotation IMGT.

7. Procédé selon l'une quelconque des revendications 1 à 6, **caractérisé en ce que** la modification d'au moins un nucléotide d'un acide nucléique se traduit par une substitution, délétion et/ou insertion d'un ou de plusieurs acides aminés du polypeptide de domaine de récepteur de cellules T codé par ledit acide nucléique.

8. Procédé selon l'une quelconque des revendications 1 à 7, **caractérisé en ce qu'**au moins un acide aminé d'au moins une région de boucle structurelle est modifié par une mutation aléatoire dirigée au site.

9. Procédé selon la revendication 8, **caractérisé en ce que** la molécule d'acide nucléique modifiée de manière aléatoire comprend au moins une unité de répétition de nucléotide ayant la séquence de codage NNS, NNN, NNK, TMT, WMT, RMC, RMG, MRT, SRC, KMT, RST, YMT, MKC, RSA, RRC, où le codage est en accord avec IUPAC.
